# EUROPEAN PATENT APPLICATION

(11) **EP 4 714 938 A1**
(43) Date of publication of application: **25.03.2026**
(21) Application number: 24806568.2
(22) Date of filing: 14.05.2024
(51) Int. Cl.: C07C 381/10, C07D 213/02, C07D 239/24, C07D 263/30, C07D 277/20, C07D 333/10, A61K 31/381, A61K 31/39, A61K 31/397, A61K 31/4164, A61K 31/421, A61K 31/44, A61K 31/501, A61K 31/505, A61P 35/00

(54) **HDAC1/2 SELECTIVE INHIBITOR AND USE THEREOF**

(30) Priority: 15.05.2023 CN 202310547297
(71) Applicant: Wigen Biomedicine Technology (Shanghai) Co., Ltd., Shanghai 201203 (CN)
(72) Inventor: XU, Ruihua, Guangzhou, Guangdong 510062 (CN); WANG, Feng, Guangzhou, Guangdong 510095 (CN); XIE, Yuli, Shanghai 201203 (CN); LIU, Wenzhong, Shanghai 201203 (CN); QIAN, Lihui, Shanghai 201203 (CN)
(74) Representative: Dr. Langfinger & Partner
(86) International application number: PCT/CN2024/093036
(87) International publication number: WO 2024/235218

(57) **Abstract**

An HDAC1/2 selective inhibitor and the use thereof. Specifically, the present invention relates to a compound as represented by general formula (1) and a preparation method therefor, and the use of the compound of general formula (1) and each isomer, each crystal form, a pharmaceutically acceptable salt, hydrate or solvate thereof as an HDAC1/2 selective inhibitor in the preparation of an anti-tumor drug.

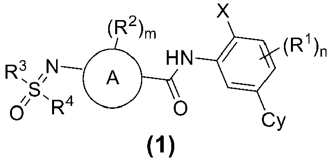

## Description

The present application claims priority to Chinese Patent Application No. 202310547297.2 filed on May 15, 2023, which is incorporated herein by reference in its entirety.

### TECHNICAL FIELD

The present disclosure pertains to the field of pharmaceutical chemistry, and particularly relates to a class of HDAC1/2 selective inhibitors, a preparation method therefor, and use of such compounds in preparing a medicament for treating or preventing a cancer.

### BACKGROUND

Histone deacetylases (HDACs) are a class of proteases, and their histone acetylation and deacetylation of the chromatin are one of the key processes in the regulation of gene expression. Abnormal gene expression is the molecular biological basis for the development of tumors and some genetic and metabolic diseases. The acetylation level of histones is coordinately controlled by histone acetyltransferases (HATs) and histone deacetylases (HDACs). When HDACs are overexpressed and recruited by transcription factors, they can lead to abnormal inhibition of specific genes, resulting in tumors and other diseases.

HDACs constitute a large enzyme family, with 18 known different subtypes currently categorized into four major classes. Class I includes four subtypes: HDAC1, HDAC2, HDAC3, and HDAC8; class II includes six subtypes: HDAC4, HDAC5, HDAC6, HDAC7, HDAC9, and HDAC10 (among which HDAC4, HDAC5, HDAC7, and HDAC9 belong to class IIa, and HDAC6 and HDAC10 belong to class IIb); class IV includes only one subtype, HDAC11, which exhibits a certain degree of homology with the previous two classes, separately; class III includes 7 subtypes (SIRT1-SIRT7) and exhibits no structural homology with the previous three classes.

According to the published clinical results, although selective inhibitors of HDAC1, HDAC2, and HDAC3, such as MS275 (entinostat), chidamide (marketed), and CXD101, have significantly lower toxic and side effects compared to pan HDAC inhibitors such as vorinostat, romidepsin, belinostat, and panobinostat, severe adverse reactions have still been observed in clinical trials. These adverse reactions include hematological adverse reactions, systemic adverse reactions (including fatigue and fever), gastrointestinal adverse reactions, metabolic and nutritional system adverse reactions, as well as other adverse reactions such as dizziness and rash.

### SUMMARY

The present disclosure provides a series of compounds with high selectivity for HDAC1/2. These compounds exhibit excellent efficacy and safety in various *in vitro* and *in vivo* evaluation experiments, and are of great clinical significance for improving the efficacy and safety of existing drugs.

Specifically, the present invention provides a compound of general formula (1) or an isomer thereof, a crystalline form thereof, a pharmaceutically acceptable salt thereof, a hydrate thereof, or a solvate thereof: 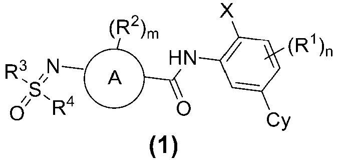 wherein in general formula (1):
X is -NH₂ or -OH;
Cy is (C6-C14) aryl or (5-14 membered) heteroaryl, wherein the (C6-C14) aryl or (5-14 membered) heteroaryl may be each independently and optionally substituted with 1, 2, 3, or 4 R⁵;
ring A is (C6-C14) aryl, (5-14 membered) heteroaryl, (C3-C14) cycloalkyl, or (3-14 membered) heterocycloalkyl;
R¹ is H, D, or halogen;
R² is H, D, halogen, (C1-C4) alkyl, (C1-C4) alkoxy, (C1-C4) haloalkyl, (C2-C4) alkenyl, (C2-C4) alkynyl, (C3-C6) cycloalkyl, or (3-6 membered) heterocycloalkyl;
R³ is (C1-C6) alkyl, (C1-C6) alkoxy, (C1-C6) haloalkyl, (C2-C4) alkenyl, (C2-C4) alkynyl, (C3-C6) cycloalkyl, or (3-6 membered) heterocycloalkyl, wherein the (C1-C6) alkyl, (C1-C6) alkoxy, (C1-C6) haloalkyl, (C2-C4) alkenyl, (C2-C4) alkynyl, (C3-C6) cycloalkyl, or (3-6 membered) heterocycloalkyl may be each independently and optionally substituted with 1, 2, 3, or 4 R^{a};
R⁴ is (C1-C6) alkyl, (C1-C6) alkoxy, (C1-C6) haloalkyl, (C2-C4) alkenyl, (C2-C4) alkynyl, (C3-C6) cycloalkyl, or (3-6 membered) heterocycloalkyl, wherein the (C1-C6) alkyl, (C1-C6) alkoxy, (C1-C6) haloalkyl, (C2-C4) alkenyl, (C2-C4) alkynyl, (C3-C6) cycloalkyl, or (3-6 membered) heterocycloalkyl may be each independently and optionally substituted with 1, 2, 3, or 4 R^{b};
or R³ and R⁴, together with the S atom to which they are attached, form (3-16 membered) heterocycloalkyl, wherein the (3-16 membered) heterocycloalkyl may be each independently and optionally substituted with 1, 2, 3, or 4 R⁶;
R⁵ is H, D, halogen, hydroxy, amino, cyano, nitro, -OR^{a}, -NR^{a}R^{b}, -C(O)R^{a}, -CO₂R^{a}, -(CH₂)mR^{a}, -(CH₂)mCO₂R^{a}, -(CH₂)ₘ-CONR^{a}R^{b}, -S(O)ₚR^{a}, -S(O)ₚNR^{a}R^{b}, -CONR^{a}R^{b}, -C(=NR^{a})-NR^{a}R^{b}, -NR^{a}COR^{b}, -CR^{a}CONR^{a}R^{b}, - NR^{a}CO₂R^{a}, -NR^{a}S(O)ₚNR^{a}R^{b}, -NR^{a}S(O)ₚR^{a}, -P(O)ₚR^{a}R^{b}, (C1-C8) alkyl, (C1-C8) alkoxy, (C1-C8) haloalkyl, (C2-C8) alkenyl, (C2-C8) alkynyl, (C3-C14) cycloalkyl, (3-14 membered) heterocycloalkyl, (C6-C14) aryl, (5-14 membered) heteroaryl, -(C1-C8) alkylene-(C3-C14) cycloalkyl, -(C1-C8) alkylene-(3-14 membered) heterocycloalkyl, -(C1-C8) alkylene-(C6-C14) aryl, or -(C1-C8) alkylene-(5-14 membered) heteroaryl, wherein the (C1-C8) alkyl, (C1-C8) alkoxy, (C1-C8) haloalkyl, (C2-C8) alkenyl, (C2-C8) alkynyl, (C3-C14) cycloalkyl, (3-14 membered) heterocycloalkyl, (C6-C14) aryl, (5-14 membered) heteroaryl, -(C1-C8) alkylene-(C3-C14) cycloalkyl, -(C1-C8) alkylene-(3-14 membered) heterocycloalkyl, -(C1-C8) alkylene-(C6-C14) aryl, or -(C1-C8) alkylene-(5-14 membered) heteroaryl may be each independently and optionally substituted with 1, 2, 3, or 4 R^{c};
R⁶ is H, D, halogen, hydroxy, amino, cyano, nitro, -OR^{a}, -NR^{a}R^{b}, -C(O)R^{a}, -COzR^{a}, -(CH₂)mR^{a}, -(CH₂)mCO₂R^{a}, -(CH₂)ₘ-CONR^{a}R^{b}, -S(O)ₚR^{a}, -S(O)ₚNR^{a}R^{b}, -CONR^{a}R^{b}, -C(=NR^{a})-NR^{a}R^{b}, -NR^{a}COR^{b}, -CR^{a}CONR^{a}R^{b}, - NR^{a}CO₂R^{a}, -NR^{a}S(O)ₚNR^{a}R^{b}, -NR^{a}S(O)ₚR^{a}, -P(O)ₚR^{a}R^{b}, (C1-C8) alkyl, (C1-C8) alkoxy, (C1-C8) haloalkyl, (C2-C8) alkenyl, (C2-C8) alkynyl, (C3-C14) cycloalkyl, (3-14 membered) heterocycloalkyl, (C6-C14) aryl, (5-14 membered) heteroaryl, -(C1-C8) alkylene-(C3-C14) cycloalkyl, -(C1-C8) alkylene-(3-14 membered) heterocycloalkyl, -(C1-C8) alkylene-(C6-C14) aryl, or -(C1-C8) alkylene-(5-14 membered) heteroaryl, wherein the (C1-C8) alkyl, (C1-C8) alkoxy, (C1-C8) haloalkyl, (C2-C8) alkenyl, (C2-C8) alkynyl, (C3-C14) cycloalkyl, (3-14 membered) heterocycloalkyl, (C6-C14) aryl, (5-14 membered) heteroaryl, -(C1-C8) alkylene-(C3-C14) cycloalkyl, -(C1-C8) alkylene-(3-14 membered) heterocycloalkyl, -(C1-C8) alkylene-(C6-C14) aryl, or -(C1-C8) alkylene-(5-14 membered) heteroaryl may be each independently and optionally substituted with 1, 2, 3, or 4 R^{c}; or when 2 R⁶ are attached to the same atom, the 2 R⁶, together with the atom to which they are attached, may form one oxo group, (C3-C6) cycloalkyl group, or (3-6 membered) heterocycloalkyl group;
R^{a} and R^{b} are each independently -H, -D, halogen, hydroxy, amino, cyano, nitro, (C1-C8) alkyl, (C1-C8) alkoxy, (C1-C8) haloalkyl, (C2-C8) alkenyl, (C2-C8) alkynyl, (C3-C14) cycloalkyl, (3-14 membered) heterocycloalkyl, (C6-C14) aryl, (5-14 membered) heteroaryl, -(C1-C8) alkylene-(C3-C14) cycloalkyl, -(C1-C8) alkylene-(3-14 membered) heterocycloalkyl, -(C1-C8) alkylene-(C6-C14) aryl, or -(C1-C8) alkylene-(5-14 membered) heteroaryl, wherein the (C1-C8) alkyl, (C1-C8) alkoxy, (C1-C8) haloalkyl, (C2-C8) alkenyl, (C2-C8) alkynyl, (C3-C14) cycloalkyl, (3-14 membered) heterocycloalkyl, (C6-C14) aryl, (5-14 membered) heteroaryl, -(C1-C8) alkylene-(C3-C14) cycloalkyl, -(C1-C8) alkylene-(3-14 membered) heterocycloalkyl, - (C1-C8) alkylene-(C6-C14) aryl, or -(C1-C8) alkylene-(5-14 membered) heteroaryl may be each independently and optionally substituted with 1, 2, 3, or 4 R^{c}; or when 2 R^{a} are attached to the same atom, the 2 R^{a} may form one oxo group; or when 2 R^{b} are attached to the same atom, the 2 R^{b} may form one oxo group; or R^{a} and R^{b} attached to the same atom, together with the atom to which they are attached, form one (5-7 membered) heterocycloalkyl group or (C3-C9) cycloalkyl group, wherein the (5-7 membered) heterocycloalkyl group or (C3-C9) cycloalkyl group is each independently and optionally substituted with 1, 2, 3, or 4 R^{c};
R^{c} is -H, -D, halogen, hydroxy, amino, cyano, nitro, (C1-C8) alkyl, (C1-C8) alkoxy, (C1-C8) haloalkyl, (C2-C8) alkenyl, (C2-C8) alkynyl, (C3-C14) cycloalkyl, (3-14 membered) heterocycloalkyl, (C6-C14) aryl, (5-14 membered) heteroaryl, -(C1-C8) alkylene-(C1-C8) alkoxy, -(C1-C8) alkylene-(C3-C14) cycloalkyl, -(C1-C8) alkylene-(3-14 membered) heterocycloalkyl, -(C1-C8) alkylene-(C6-C14) aryl, or -(C1-C8) alkylene-(5-14 membered) heteroaryl; or when 2 R^{c} are attached to the same atom, the 2 R^{c} may form one oxo group, (C3-C6) cycloalkyl group, or (3-6 membered) heterocycloalkyl group;
m, n, and p are integers of 0, 1, or 2.

In another preferred embodiment, in general formula (1), X is -NH₂ or -OH; preferably, X is -NH₂.

In another preferred embodiment, in general formula (1), ring A is (C6-C10) aryl, (5-10 membered) heteroaryl, (C3-C12) cycloalkyl, or (3-12 membered) heterocycloalkyl; preferably, ring A is phenyl, (5-7 membered) heteroaryl, or (C5-C7) cycloalkyl; more preferably, ring A is phenyl, pyridinyl, or cyclohexyl.

In another preferred embodiment, in general formula (1), Cy is (C6-C10) aryl or (5-12 membered) heteroaryl, wherein the (C6-C10) aryl or (5-12 membered) heteroaryl may be each independently and optionally substituted with 1, 2, 3, or 4 R⁵; preferably, Cy is phenyl or (5-7 membered) heteroaryl.

In another preferred embodiment, in general formula (1), Cy is phenyl or (5-6 membered) heteroaryl, preferably

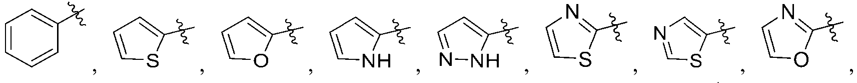

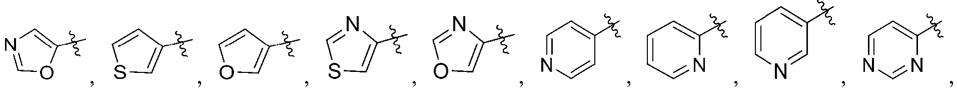

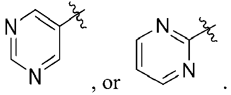

In another preferred embodiment, in general formula (1), R⁵ is H, D, halogen, hydroxy, amino, cyano, nitro, - OR^{a}, -NR^{a}R^{b}, -C(O)R^{a}, -CO₂R^{a}, -(CH₂)ₘR^{a}, -(CH₂)ₘCO₂R^{a}, -(CH₂)ₘCONR^{a}R^{b}, -S(O)ₚR^{a}, -S(O)ₚNR^{a}R^{b}, - CONR^{a}R^{b}, -C(=NR^{a})-NR^{a}R^{b}, -NR^{a}COR^{b}, -CR^{a}CONR^{a}R^{b}, -NR^{a}CO₂R^{a}, -NR^{a}S(O)ₚNR^{a}R^{b}, -NR^{a}S(O)ₚR^{a}, - P(O)ₚR^{a}R^{b}, (C1-C6) alkyl, (C1-C6) alkoxy, (C1-C6) haloalkyl, (C2-C6) alkenyl, (C2-C6) alkynyl, (C3-C12) cycloalkyl, (3-12 membered) heterocycloalkyl, (C6-C14) aryl, (5-14 membered) heteroaryl, -(C1-C6) alkylene-(C3-C14) cycloalkyl, -(C1-C6) alkylene-(3-14 membered) heterocycloalkyl, -(C1-C6) alkylene-(C6-C14) aryl, or -(C1-C6) alkylene-(5-14 membered) heteroaryl, wherein the (C1-C6) alkyl, (C1-C6) alkoxy, (C1-C6) haloalkyl, (C2-C6) alkenyl, (C2-C6) alkynyl, (C3-C12) cycloalkyl, (3-12 membered) heterocycloalkyl, (C6-C14) aryl, (5-14 membered) heteroaryl, -(C1-C6) alkylene-(C3-C14) cycloalkyl, -(C1-C6) alkylene-(3-14 membered) heterocycloalkyl, -(C1-C6) alkylene-(C6-C14) aryl, or -(C1-C6) alkylene-(5-14 membered) heteroaryl may be each independently and optionally substituted with 1, 2, 3, or 4 R^{c}; preferably, R⁵ is H, D, halogen, hydroxy, amino, cyano, nitro, -OR^{a}, -NR^{a}R^{b}, -CONR^{a}R^{b}, -P(O)ₚR^{a}R^{b}, -S(O)ₚR^{a}, (C1-C4) alkyl, (C1-C4) alkoxy, (C1-C4) haloalkyl, (C2-C4 alkenyl, (C2-C4) alkynyl, (C3-C7) cycloalkyl, or (3-7 membered) heterocycloalkyl.

In another preferred embodiment, in general formula (1), R⁵ is H, D, F, Cl, -CN, (C1-C3) alkyl, (C1-C3) alkoxy, (C1-C3) haloalkyl, or (C3-C6) cycloalkyl; preferably, R⁵ is H, D, F, Cl, -CN, -CH₃, -CF₃, -OCH₃, or

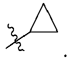

In another preferred embodiment, in general formula (1), R¹ is H, D, F, Cl, Br, or I; preferably, R¹ is H, D, or F.

In another preferred embodiment, in general formula (1), R² is H, D, F, Cl, Br, I, (C1-C3) alkyl, (C1-C3) alkoxy, (C1-C3) haloalkyl, (C2-C4) alkenyl, (C2-C4) alkynyl, (C3-C6) cycloalkyl, or (3-6 membered) heterocycloalkyl; preferably, R² is H, D, or F.

In another preferred embodiment, in general formula (1), R³ is (C1-C4) alkyl, (C1-C4) alkoxy, (C1-C4) haloalkyl, (C2-C4) alkenyl, (C2-C4) alkynyl, (C3-C6) cycloalkyl, or (3-6 membered) heterocycloalkyl, wherein the (C1-C4) alkyl, (C1-C4) alkoxy, (C1-C4) haloalkyl, (C2-C4) alkenyl, (C2-C4) alkynyl, (C3-C6) cycloalkyl, or (3-6 membered) heterocycloalkyl may be each independently and optionally substituted with 1, 2, 3, or 4 R^{a}; preferably, R³ is (C1-C4) alkyl, (C1-C4) alkoxy, or (C1-C4) haloalkyl; more preferably, R³ is methyl, ethyl, or methoxymethyl.

In another preferred embodiment, in general formula (1), R⁴ is (C1-C4) alkyl, (C1-C4) alkoxy, (C1-C4) haloalkyl, (C2-C4) alkenyl, (C2-C4) alkynyl, (C3-C6) cycloalkyl, or (3-6 membered) heterocycloalkyl, wherein the (C1-C4) alkyl, (C1-C4) alkoxy, (C1-C4) haloalkyl, (C2-C4) alkenyl, (C2-C4) alkynyl, (C3-C6) cycloalkyl, or (3-6 membered) heterocycloalkyl may be each independently and optionally substituted with 1, 2, 3, or 4 R^{b}; preferably, R⁴ is (C1-C4) alkyl, (C1-C4) alkoxy, or (C1-C4) haloalkyl; more preferably, R⁴ is methyl, ethyl, or methoxymethyl.

In another preferred embodiment, in general formula (1), R³ and R⁴, together with the S atom to which they are attached, form (3-14 membered) heterocycloalkyl, wherein the (3-14 membered) heterocycloalkyl may be each independently and optionally substituted with 1, 2, 3, or 4 R⁶.

In another preferred embodiment, in general formula (1), R³ and R⁴, together with the S atom to which they are attached, form one (4-6 membered) monocyclic heterocycloalkyl group containing 1 or 2 atoms optionally selected from N, S, and O, or (7-9 membered) bicyclic spiro heterocycloalkyl group containing 1 or 2 atoms optionally selected from N, S, and O, wherein the (4-6 membered) monocyclic heterocycloalkyl group or (7-9 membered) bicyclic spiro heterocycloalkyl group may be each independently and optionally substituted with 1, 2, 3, or 4 R⁶.

In another preferred embodiment, in general formula (1), R⁶ is H, D, halogen, hydroxy, amino, cyano, nitro, - OR^{a}, -NR^{a}R^{b}, -C(O)R^{a}, -CO₂R^{a}, -(CH₂)mR^{a}, -(CH₂)ₘCO₂R^{a}, -(CH₂)ₘCONR^{a}R^{b}, -S(O)ₚR^{a}, -S(O)ₚNR^{a}R^{b}, - CONR^{a}R^{b}, -C(=NR^{a})-NR^{a}R^{b}, -NR^{a}COR^{b}, -CR^{a}CONR^{a}R^{b}, -NR^{a}CO₂R^{a}, -NR^{a}S(O)ₚNR^{a}R^{b}, -NR^{a}S(O)ₚR^{a}, - P(O)ₚR^{a}R^{b}, (C1-C6) alkyl, (C1-C6) alkoxy, (C1-C6) haloalkyl, (C2-C6) alkenyl, (C2-C6) alkynyl, (C3-C14) cycloalkyl, (3-14 membered) heterocycloalkyl, (C6-C14) aryl, (5-14 membered) heteroaryl, -(C1-C6) alkylene-(C3-C14) cycloalkyl, -(C1-C6) alkylene-(3-14 membered) heterocycloalkyl, -(C1-C6) alkylene-(C6-C14) aryl, or -(C1-C6) alkylene-(5-14 membered) heteroaryl, wherein the (C1-C6) alkyl, (C1-C6) alkoxy, (C1-C6) haloalkyl, (C2-C6) alkenyl, (C2-C6) alkynyl, (C3-C14) cycloalkyl, (3-14 membered) heterocycloalkyl, (C6-C14) aryl, (5-14 membered) heteroaryl, -(C1-C6) alkylene-(C3-C14) cycloalkyl, -(C1-C6) alkylene-(3-14 membered) heterocycloalkyl, -(C1-C6) alkylene-(C6-C14) aryl, or -(C1-C6) alkylene-(5-14 membered) heteroaryl may be each independently and optionally substituted with 1, 2, 3, or 4 R^{c}; preferably, R⁶ is H, D, halogen, hydroxy, amino, cyano, -OR^{a}, -NR^{a}R^{b}, (C1-C4) alkyl, (C1-C4) alkoxy, (C1-C4) haloalkyl, -(C1-C4) alkylene-(C3-C7) cycloalkyl, -(C1-C4) alkylene-(3-7 membered) heterocycloalkyl, or -(C1-C4) alkylene-(5-7 membered) heteroaryl, wherein the (C1-C4) alkyl, (C1-C4) alkoxy, (C1-C4) haloalkyl, -(C1-C4) alkylene-(C3-C7) cycloalkyl, -(C1-C4) alkylene-(3-7 membered) heterocycloalkyl, or -(C1-C4) alkylene-(5-7 membered) heteroaryl may be each independently and optionally substituted with 1, 2, 3, or 4 R^{c}; or when 2 R⁶ are attached to the same atom, the 2 R⁶, together with the atom to which they are attached, may form one oxo group.

In another preferred embodiment, in general formula (1), R⁶ is H, D, F, Cl, hydroxy, amino, cyano, methyl, ethyl, -NH(CH₃), -N(CH₃)₂, -CD₃, -C(O)CH₃, or

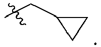

In another preferred embodiment, in general formula (1), R^{a} and R^{b} are each independently -H, -D, halogen, hydroxy, amino, cyano, nitro, (C1-C6) alkyl, (C1-C6) alkoxy, (C1-C6) haloalkyl, (C2-C6) alkenyl, (C2-C6) alkynyl, (C3-C14) cycloalkyl, (3-14 membered) heterocycloalkyl, (C6-C14) aryl, (5-14 membered) heteroaryl, -(C1-C6) alkylene-(C3-C14) cycloalkyl, -(C1-C6) alkylene-(3-14 membered) heterocycloalkyl, -(C1-C6) alkylene-(C6-C14) aryl, or -(C1-C6) alkylene-(5-14 membered) heteroaryl, wherein the (C1-C6) alkyl, (C1-C6) alkoxy, (C1-C6) haloalkyl, (C2-C6) alkenyl, (C2-C6) alkynyl, (C3-C14) cycloalkyl, (3-14 membered) heterocycloalkyl, (C6-C14) aryl, (5-14 membered) heteroaryl, -(C1-C6) alkylene-(C3-C14) cycloalkyl, -(C1-C6) alkylene-(3-14 membered) heterocycloalkyl, -(C1-C6) alkylene-(C6-C14) aryl, or -(C1-C6) alkylene-(5-14 membered) heteroaryl may be each independently and optionally substituted with 1, 2, 3, or 4 R^{c}; or when 2 R^{a} are attached to the same atom, the 2 R^{a} may form one oxo group; or when 2 R^{d} are attached to the same atom, the 2 R^{B} may form one oxo group; preferably, R^{a} and R^{b} are each independently H, (C1-C4) alkyl, (C1-C4) alkoxy, (C1-C4) haloalkyl, (C3-C7) cycloalkyl, (3-7 membered) heterocycloalkyl, (C6-C10) aryl, or (5-7 membered) heteroaryl, wherein the (C1-C4) alkyl, (C1-C4) alkoxy, (C1-C4) haloalkyl, (C3-C7) cycloalkyl, (3-7 membered) heterocycloalkyl, (C6-C10) aryl, or (5-7 membered) heteroaryl may be each independently and optionally substituted with 1, 2, 3, or 4 R^{c}.

In another preferred embodiment, in general formula (1), R^{a} and R^{b} attached to the same atom, together with the atom to which they are attached, form one (5-7 membered) heterocycloalkyl group or (C3-C9) cycloalkyl group, wherein the (5-7 membered) heterocycloalkyl group or (C3-C9) cycloalkyl group is each independently and optionally substituted with 1, 2, 3, or 4 R^{c}.

In another preferred embodiment, in general formula (1), R^{c} is -H, -D, halogen, hydroxy, amino, cyano, nitro, (C1-C6) alkyl, (C1-C6) alkoxy, (C1-C6) haloalkyl, (C2-C6) alkenyl, (C2-C6) alkynyl, (C3-C14) cycloalkyl, (3-14 membered) heterocycloalkyl, (C6-C14) aryl, (5-14 membered) heteroaryl, -(C1-C6) alkylene-(C1-C8) alkoxy, -(C1-C6) alkylene-(C3-C14) cycloalkyl, -(C1-C6) alkylene-(3-14 membered) heterocycloalkyl, -(C1-C6) alkylene-(C6-C14) aryl, or -(C1-C6) alkylene-(5-14 membered) heteroaryl; or when 2 R^{c} are attached to the same atom, the 2 R^{c} may form one oxo group; preferably, R^{c} is H, D, halogen, hydroxy, amino, cyano, (C1-C4) alkyl, (C1-C4) alkoxy, (C1-C4) haloalkyl, (C3-C7) cycloalkyl, or (3-7 membered) heterocycloalkyl.

In various different embodiments of the present invention, the compound of general formula (1) has one of the following structures:

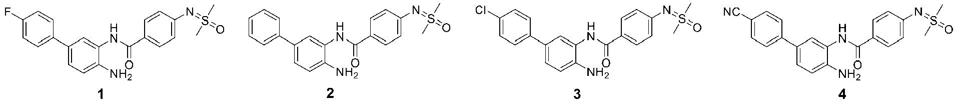

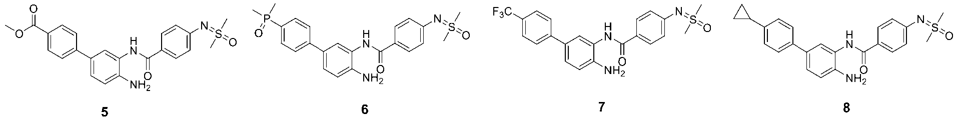

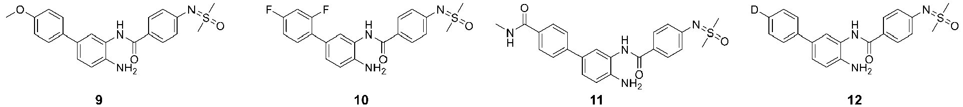

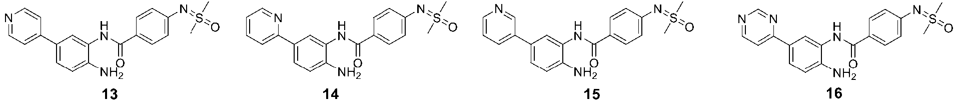

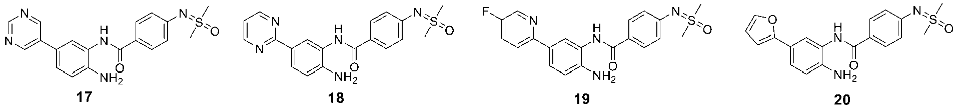

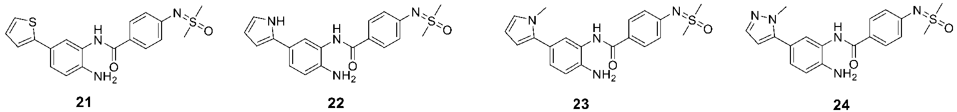

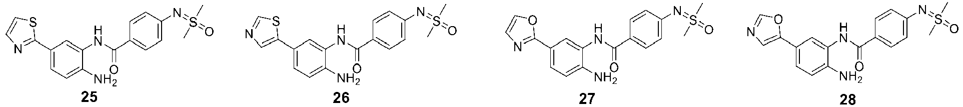

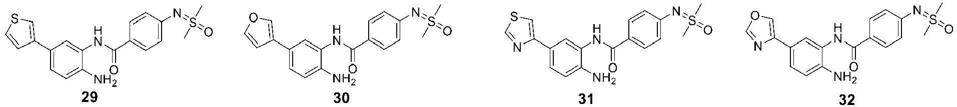

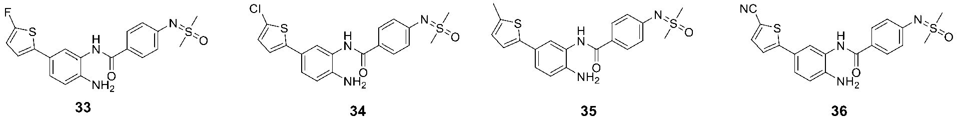

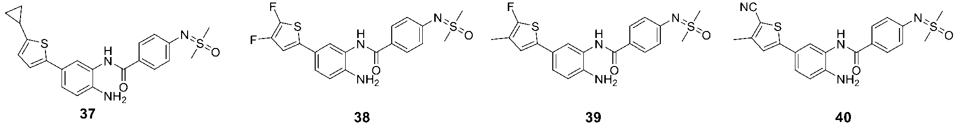

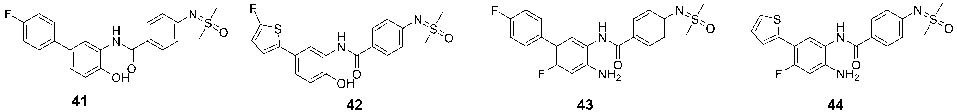

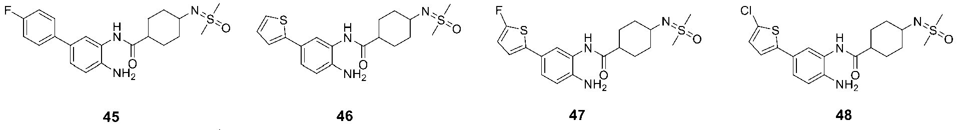

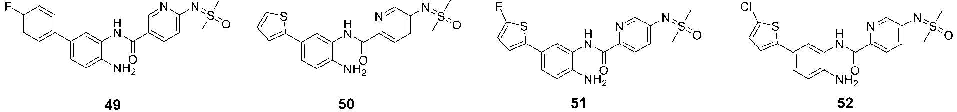

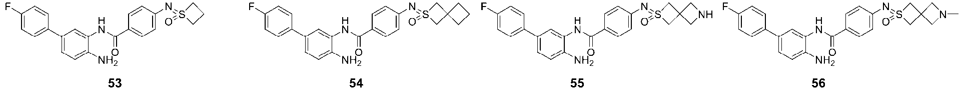

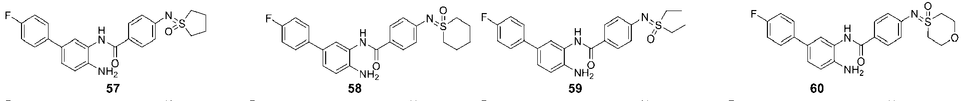

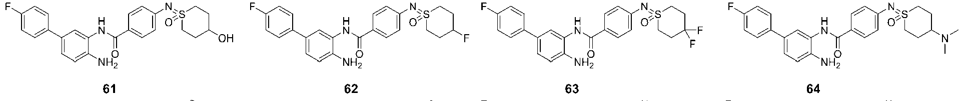

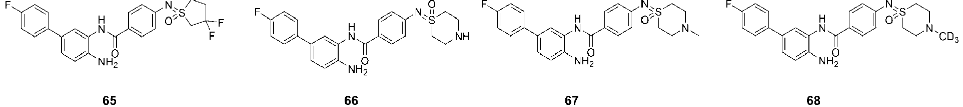

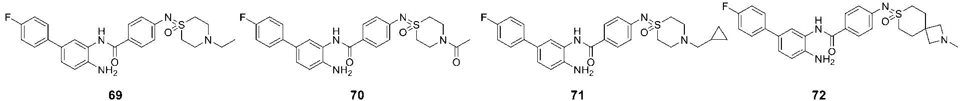

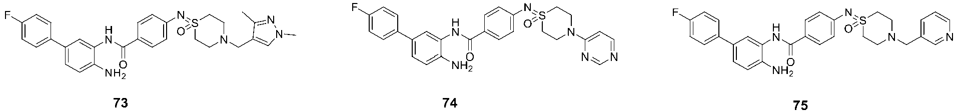

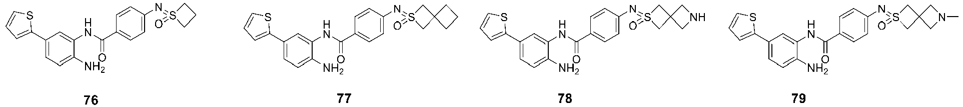

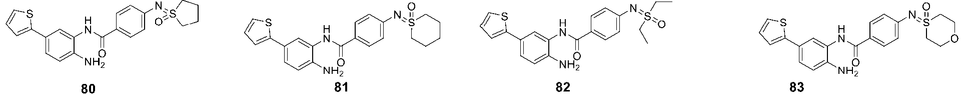

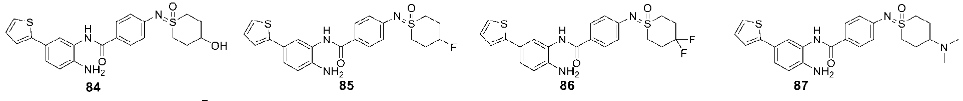

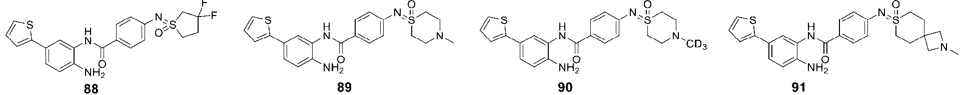

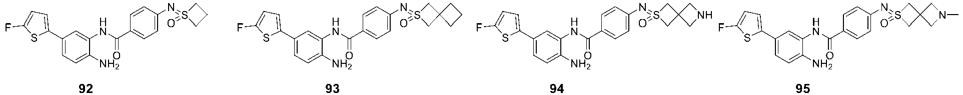

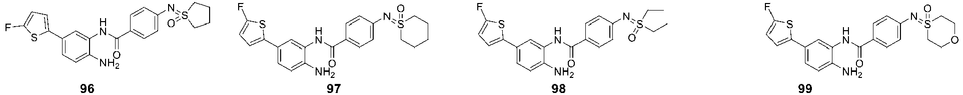

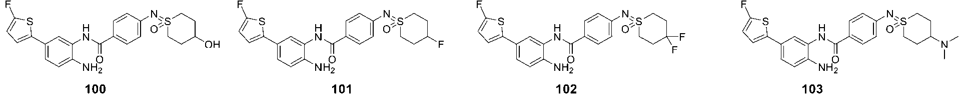

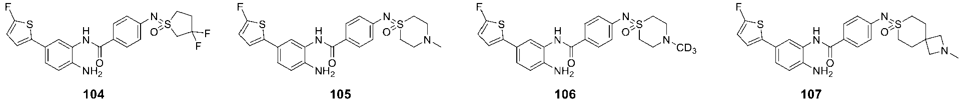

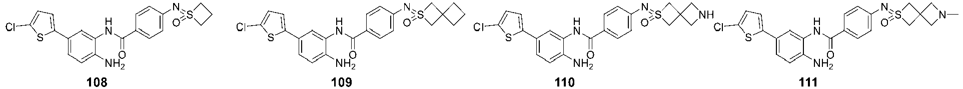

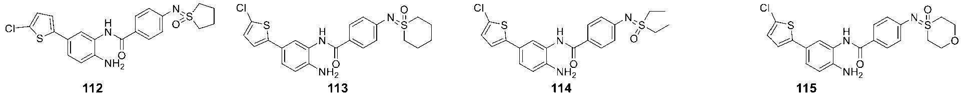

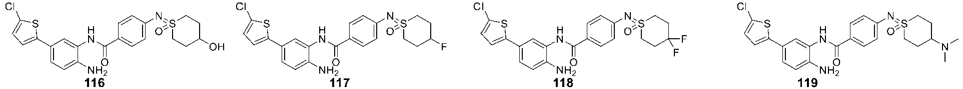

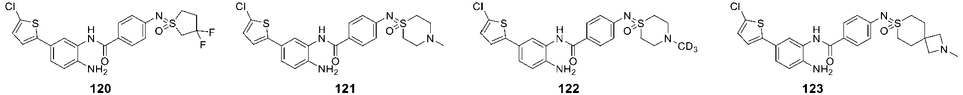

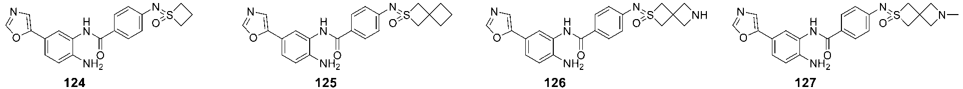

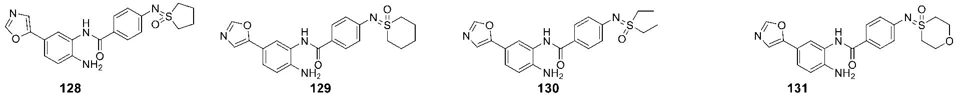

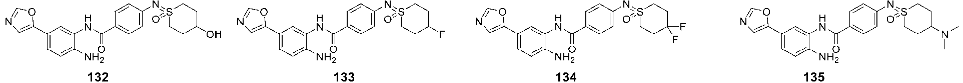

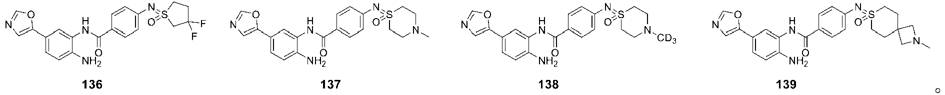

The present invention is further intended to provide a pharmaceutical composition comprising a pharmaceutically acceptable carrier, diluent, and/or excipient, as well as the compound of general formula (1) or the isomer thereof, the crystalline form thereof, the pharmaceutically acceptable salt thereof, the hydrate thereof, or the solvate thereof of the present invention as an active ingredient.

The present invention is still further intended to provide use of the compound of general formula (1) or the isomer thereof, the crystalline form thereof, the pharmaceutically acceptable salt thereof, the hydrate thereof, or the solvate thereof of the present invention or the pharmaceutical composition described above in preparing a medicament for treating, regulating, or preventing a disease related to HDAC1/2, wherein the disease is preferably cancer, and the cancer is a hematologic cancer or a solid tumor.

It should be understood that both the aforementioned general description and the following detailed description of the present invention are exemplary and explanatory, and are intended to provide further explanation of the present invention claimed.

### Synthesis of Compounds

Methods for preparing the compound of general formula (1) of the present disclosure are specifically described below, but these specific methods do not limit the present disclosure in any way.

The compound of general formula (1) described above may be synthesized using standard synthetic techniques or well-known techniques in combination with the methods described herein. In addition, the solvents, temperatures, and other reaction conditions mentioned herein may vary. Starting materials for the synthesis of the compounds may be obtained synthetically or commercially. The compounds described herein and other related compounds with different substituents may be synthesized using well-known techniques and starting materials, including the methods found in March, ADVANCED ORGANIC CHEMISTRY, 4th Ed., (Wiley 1992); Carey and Sundberg, ADVANCED ORGANIC CHEMISTRY, 4th Ed., Vols. A and B (Plenum 2000, 2001); and Green and Wuts, PROTECTIVE GROUPS IN ORGANIC SYNTHESIS, 3rd Ed., (Wiley 1999). General methods for preparing the compounds may be altered by using appropriate reagents and conditions for introducing different groups into the molecular formulas provided herein.

In one aspect, the compounds described herein are prepared according to methods well known in the art. However, the conditions of the methods, such as reactants, solvents, bases, the amount of the compound used, reaction temperature, and time required for the reaction are not limited to the following explanation. The compounds of the present disclosure may also be conveniently prepared by optionally combining various synthetic methods described herein or known in the art, and such combinations may be easily determined by those skilled in the art to which the present disclosure pertains. In one aspect, the present disclosure further provides a method for preparing the compound of general formula (1), which is prepared using general reaction scheme 1 - 3 below:

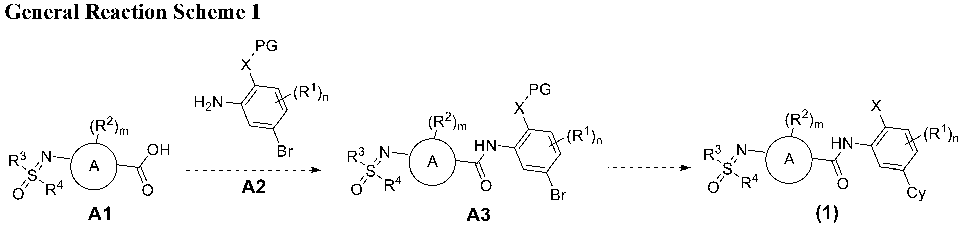

A, Cy, R¹, R², R³, R⁴, X, m, and n are as defined above, and PG represents a commonly used amine group or hydroxyl-protecting group (e.g., Boc, Cbz, Bz, Fmoc, or Bn). As shown in general reaction scheme 1, compound A1 and starting material A2 are subjected to a condensation reaction to give compound A3, and A3 is subjected to a coupling reaction and a conventional deprotection reaction to give the target compound (1).

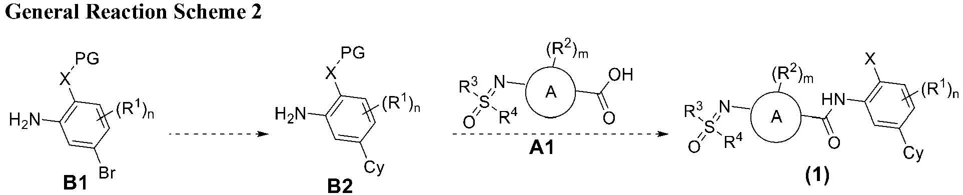

A, Cy, R¹, R², R³, R⁴, X, m, and n are as defined above, and PG represents a commonly used amine group or hydroxyl-protecting group (e.g., Boc, Cbz, Bz, Fmoc, or Bn). As shown in general reaction scheme 2, compound B1 is subjected to a coupling reaction to give compound B2, and compound B2 and starting material A1 are subjected to a condensation reaction and a conventional deprotection reaction to give the target compound (1).

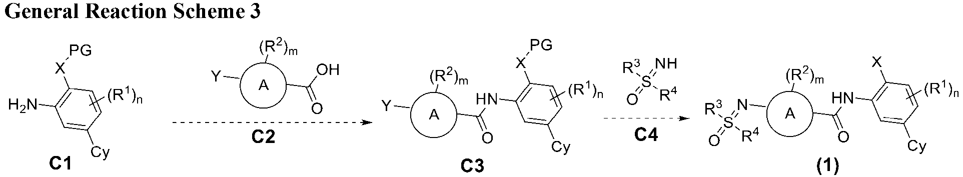

A, Cy, R¹, R², R³, R⁴, X, m, and n are as defined above, PG represents a commonly used amine group or hydroxyl-protecting group (e.g., Boc, Cbz, Bz, Fmoc, or Bn), and Y represents a leaving group such as Br, I, or OTf. As shown in general reaction scheme 3, compound C1 and starting material C2 are subjected to a condensation reaction to give the target compound C3, and compound C3 and compound C4 are subjected to a coupling reaction and a conventional deprotection reaction to give the target compound (1).

### Further Forms of Compounds

"Pharmaceutically acceptable" herein refers to a substance, such as a carrier or diluent, which will not lead to loss of biological activity or properties of a compound and is relatively non-toxic. For example, when an individual is given a substance, the substance will not cause undesired biological effects or interact with any component contained therein in a deleterious manner.

The term "pharmaceutically acceptable salt" refers to a form of a compound that does not cause significant irritation to the organism receiving the administration or eliminate the biological activity and properties of the compound. In certain specific aspects, the pharmaceutically acceptable salt is obtained by subjecting the compound of the general formula to a reaction with acids or bases, wherein the acids or bases include, but are not limited to, those found in Stahl and Wermuth, Handbook of Pharmaceutical Salts: Properties, Selection, and Use, 1st Ed., (Wiley, 2002).

It should be understood that references to pharmaceutically acceptable salts include solvent addition forms or crystalline forms, especially solvates or polymorphs. A solvate contains either stoichiometric or nonstoichiometric amount of solvent and is selectively formed during crystallization in a pharmaceutically acceptable solvent such as water and ethanol. Hydrates are formed when the solvent is water, or alcoholates are formed when the solvent is ethanol. The solvates of the compound of general formula (1) are conveniently prepared or formed according to the methods described herein. For example, hydrates of the compound of general formula (1) are conveniently prepared by recrystallization in a mixed solvent of water/organic solvent, wherein the organic solvent used includes, but is not limited to, tetrahydrofuran, acetone, ethanol, or methanol. Furthermore, the compounds described herein may be present in either a non-solvated form or a solvated form. In general, the solvated forms are considered equivalent to the non-solvated forms for purposes of the compounds and methods provided herein.

In other specific examples, the compound of general formula (1) is prepared in different forms including, but not limited to, amorphous, pulverized, and nanoparticle forms. In addition, the compound of general formula (1) includes crystalline forms, and may also be polymorphs. Polymorphs include different lattice arrangements of the same elements of a compound. Polymorphs generally have different X-ray diffraction spectra, infrared spectra, melting points, density, hardness, crystalline forms, optical and electrical properties, stability, and solubility. Different factors such as a recrystallization solvent, crystallization rate, and storage temperature may lead to a single dominant crystalline form.

In another aspect, the compound of general formula (1) may have a chiral center and/or axial chirality, and thus may be present in the form of a racemate, a racemic mixture, a single enantiomer, a diastereomeric compound, a single diastereomer, and a cis-trans isomer. Each chiral center or axial chirality will independently produce two optical isomers, and all possible optical isomers, diastereomeric mixtures, and pure or partially pure compounds are included within the scope of the present disclosure. The present disclosure is meant to include all such isomeric forms of these compounds.

The compound of the present disclosure may contain unnatural proportions of atomic isotopes at one or more of the atoms that constitute the compound. For example, the compound may be labeled with radioactive isotopes, such as tritium (³H), iodine-125 (¹²⁵I), and C-14 (¹⁴C). For another example, deuterium can be used to substitute a hydrogen atom to form a deuterated compound. The bond formed by deuterium and carbon is stronger than that formed by ordinary hydrogen and carbon. Compared with an undeuterated medicament, the deuterated medicament generally has the advantages of reduced toxic and side effects, increased pharmaceutical stability, enhanced efficacy, prolonged pharmaceutical *in vivo* half-life, and the like. All isotopic variations of the compound of the present disclosure, whether radioactive or not, are contained within the scope of the present disclosure.

Any atom of the compound of the present disclosure, unless otherwise specified, refers to an isotope of the atom in stable state of the compound. Unless otherwise specified, when a site in a molecular structure is selected as "H" or "hydrogen", the site should be understood as having the natural abundance of the hydrogen isotope. Similarly, unless otherwise specified, when a site is selected as "D" or "deuterium", the site should be understood to have a deuterium isotopic abundance that is at least 3000 times its natural abundance (the natural abundance of the deuterium isotope is 0.015%).

More preferably, each deuterated site of the deuterated compounds of the present disclosure has a deuterium atom abundance that is at least 3500 times its natural abundance (52.2% deuterium atom enrichment). More preferably, the deuterium atom abundance is at least 4500 times the natural abundance (67.5% deuterium atom enrichment). More preferably, the deuterium atom abundance is at least 5000 times the natural abundance (75% deuterium atom enrichment). More preferably, the deuterium atom abundance is at least 6000 times the natural abundance (90% deuterium atom enrichment). More preferably, the deuterium atom abundance is at least 6333 times the natural abundance (95% deuterium atom enrichment). More preferably, the deuterium atom abundance is at least 6466.7 times the natural abundance (97% deuterium atom enrichment). More preferably, the deuterium atom abundance is at least 6600 times the natural abundance (99% deuterium atom enrichment). More preferably, the deuterium atom abundance is at least 6633.3 times the natural abundance (99.5% deuterium atom enrichment).

### Terminology

Unless otherwise stated, the terms used in the present application, including those in the specification and claims, are defined as follows. It must be noted that in the specification and the appended claims, the singular forms "a" and "an" include plural meanings unless clearly indicated otherwise. Unless otherwise stated, conventional methods for mass spectrometry, nuclear magnetic resonance spectroscopy, HPLC, protein chemistry, biochemistry, recombinant DNA techniques, and pharmacology are used. As used herein, "or" or "and" refers to "and/or" unless otherwise stated.

Unless otherwise specified, "alkyl" refers to a saturated aliphatic hydrocarbon group, including linear and branched groups containing 1 to 6 carbon atoms. Lower alkyl groups containing 1 to 4 carbon atoms, such as methyl, ethyl, propyl, 2-propyl, n-butyl, isobutyl, and tert-butyl, are preferred. Lower alkyl groups containing 1 to 3 carbon atoms, such as methyl, ethyl, propyl, and 2-propyl, are further preferred. As used herein, "alkyl" includes unsubstituted and substituted alkyl, particularly alkyl substituted with one or more halogens. Preferred alkyl is selected from CH₃, CH₃CH₂, CF₃, CHF₂, CF₃CH₂, CF₃(CH₃)CH, ⁱPr, ⁿPr, ⁱBu, ⁿBu, and ^{t}Bu.

Unless otherwise specified, "alkylene" refers to a divalent alkyl as defined above. Examples of alkylene include, but are not limited to, methylene and ethylene.

Unless otherwise specified, "alkenyl" refers to an unsaturated aliphatic hydrocarbon group containing carboncarbon double bonds, including linear or branched groups containing 1 to 14 carbon atoms. Lower alkenyl groups containing 1 to 4 carbon atoms, such as vinyl, 1-propenyl, 1-butenyl, or 2-methylpropenyl, are preferred. Lower alkenyl groups containing 1 to 2 carbon atoms are further preferred.

Unless otherwise specified, "alkenylene" refers to a divalent alkenyl as defined above.

Unless otherwise specified, "alkynyl" refers to an unsaturated aliphatic hydrocarbon group containing carboncarbon triple bonds, including linear and branched groups containing 1 to 14 carbon atoms. Lower alkynyl groups containing 1 to 4 carbon atoms, such as ethynyl, 1-propynyl, or 1-butynyl, are preferred. Lower alkynyl groups containing 1 to 2 carbon atoms are further preferred.

Unless otherwise specified, "alkynylene" refers to a divalent alkynyl as defined above.

Unless otherwise specified, "cycloalkyl" refers to a non-aromatic hydrocarbon ring system (monocyclic, bicyclic, or polycyclic), and is preferably a non-aromatic hydrocarbon ring system containing 3 to 14 ring carbon atoms (C₃₋₁₄ cycloalkyl). In some embodiments, cycloalkyl has 3 to 10 ring carbon atoms (C₃₋₁₀ cycloalkyl). In some embodiments, cycloalkyl has 3 to 8 ring carbon atoms (C₃₋₈ cycloalkyl). In some embodiments, cycloalkyl has 3 to 7 ring carbon atoms (C₃₋₇ cycloalkyl). In some embodiments, cycloalkyl has 3 to 6 ring carbon atoms (C₃₋₆ cycloalkyl). In some embodiments, cycloalkyl has 4 to 6 ring carbon atoms (C₄₋₆ cycloalkyl). In some embodiments, cycloalkyl has 5 to 6 ring carbon atoms (C₅₋₆ cycloalkyl). In some embodiments, cycloalkyl has 5 to 10 ring carbon atoms (C₅₋₁₀ cycloalkyl). For cycloalkyl, partially unsaturated cycloalkyl may be referred to as "cycloalkenyl" if the carbocyclic ring contains at least one double bond, or "cycloalkynyl" if the carbocyclic ring contains at least one triple bond. Cycloalkyl may include monocyclic or polycyclic groups (e.g., having 2, 3, or 4 fused rings) and spiro rings. In some embodiments, cycloalkyl is monocyclic. In some embodiments, cycloalkyl is bicyclic. In some embodiments, cycloalkyl is monocyclic or bicyclic. In some embodiments, cycloalkyl is tricyclic. The ring carbon atoms of cycloalkyl may optionally be oxidized to form an oxo or thio group. Cycloalkyl further includes cycloalkylene. In some embodiments, cycloalkyl contains 0, 1, or 2 double bonds. In some embodiments, cycloalkyl contains 1 or 2 double bonds (partially unsaturated cycloalkyl). In some embodiments, cycloalkyl may be fused to aryl, heteroaryl, cycloalkyl, and heterocycloalkyl. In some embodiments, cycloalkyl may be fused to aryl, cycloalkyl, and heterocycloalkyl. In some embodiments, cycloalkyl may be fused to aryl and heterocycloalkyl. In some embodiments, cycloalkyl may be fused to aryl and cycloalkyl. Examples of cycloalkyl include cyclopropyl, cyclobutyl, cyclopentyl, cyclohexyl, cycloheptyl, cyclopentenyl, cyclohexenyl, cyclohexadienyl, cycloheptatrienyl, norcamphanyl, norpinanyl, norcamyl, bicyclo[1.1.1]pentyl, bicyclo[2.1.1]hexyl, and the like.

Unless otherwise specified, "cycloalkylene" refers to a divalent cycloalkyl as defined above.

Unless otherwise specified, "alkoxy" refers to an alkyl group that bonds to the rest of the molecule through an ether oxygen atom. Representative alkoxy groups are those having 1 to 6 carbon atoms, such as methoxy, ethoxy, propoxy, isopropoxy, butoxy, isobutoxy, sec-butoxy, and tert-butoxy. As used herein, "alkoxy" includes unsubstituted and substituted alkoxy, particularly alkoxy substituted with one or more halogens. Preferred alkoxy is selected from OCH₃, OCF₃, CHF₂O, CF₃CH₂O, ⁱ⁻PrO, ⁿ⁻PrO, ⁱ⁻BuO, ⁿ⁻BuO, and ^{t-}BuO.

Unless otherwise specified, "aryl" refers to an aromatic hydrocarbon group, which is monocyclic or polycyclic; for example, a monocyclic aryl ring is fused to one or more carbocyclic aromatic groups. Examples of aryl include, but are not limited to, phenyl, naphthyl, and phenanthryl.

Unless otherwise specified, "aryloxy" refers to an aryl group that bonds to the rest of the molecule through an ether oxygen atom. Examples of aryloxy include, but are not limited to, phenoxy and naphthoxy.

Unless otherwise specified, "arylene" refers to a divalent aryl as defined above. Examples of arylene include, but are not limited to, phenylene, naphthylene, and phenanthrylene.

Unless otherwise specified, "heteroaryl" refers to a substituted or unsubstituted aromatic group containing one or more heteroatoms independently selected from O, N, and S, wherein the number of heteroatoms is preferably 1, 2, 3, or 4; preferably, the heteroaryl is a 5- to 14-membered aromatic group containing 1 to 4 heteroatoms selected from oxygen, sulfur, and nitrogen; more preferably, the heteroaryl is a 5- to 9-membered aromatic group containing 1 to 2 heteroatoms optionally selected from oxygen, sulfur, and nitrogen; more preferably, the heteroaryl is a 5- to 6-membered aromatic group containing 1 to 3 heteroatoms optionally selected from oxygen, sulfur, and nitrogen. Heteroaryl is monocyclic or polycyclic. Monocyclic heteroaryl is preferably a 5- to 6-membered aromatic group containing 1 to 3 heteroatoms optionally selected from oxygen, nitrogen, and sulfur. More preferably, monocyclic heteroaryl is a 5- to 6-membered aromatic group containing 1 to 2 heteroatoms optionally selected from oxygen, nitrogen, and sulfur. More preferably, monocyclic heteroaryl is a 5- to 6-membered aromatic group containing 1 heteroatom optionally selected from oxygen, nitrogen, and sulfur. In some embodiments, a monocyclic heteroaryl ring is fused to one or more carbocyclic aromatic groups or other monocyclic heterocycloalkyl groups. Examples of heteroaryl include, but are not limited to, pyridinyl, pyridazinyl, imidazolyl, pyrimidinyl, pyrazolyl, triazolyl, pyrazinyl, quinolinyl, isoquinolinyl, furanyl, thienyl, isoxazolyl, thiazolyl, oxazolyl, 1,2,4-oxadiazolyl, 1,3,4-oxadiazolyl, 1,2,5-oxadiazolyl, isothiazolyl, pyrrolyl, indolyl, benzimidazolyl, benzofuranyl, benzothiazolyl, benzothienyl, benzoxazolyl, benzopyridinyl, pyrrolopyrimidinyl, 1H-pyrrolo[3,2-b]pyridinyl, 1H-pyrrolo[2,3-c]pyridinyl, 1H-pyrrolo[3,2-c]pyridinyl, 1H-pyrrolo[2,3-b]pyridinyl, 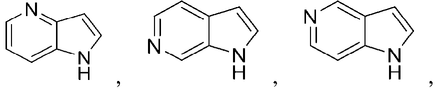 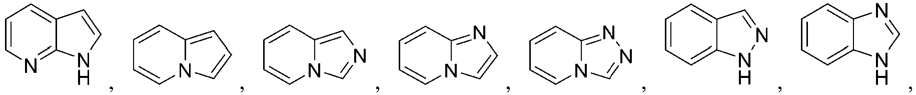 and

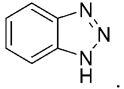

Unless otherwise specified, "heteroarylene" refers to a divalent heteroaryl as defined above.

Unless otherwise specified, "heterocycloalkyl" refers to a non-aromatic ring or ring system, which may optionally contain one or more alkenylene as part of the ring structure, having at least one heteroatom ring member independently selected from boron, phosphorus, nitrogen, sulfur, oxygen, and selenium; heterocycloalkyl is preferably a saturated or partially unsaturated ring containing 1 to 4 heteroatoms selected from oxygen, sulfur, and nitrogen, more preferably a saturated or partially unsaturated ring containing 1 to 2 heteroatoms selected from oxygen, sulfur, and nitrogen. In some embodiments, heterocycloalkyl is a 5- to 8-membered non-aromatic ring containing ring carbon atoms and 1 to 4 ring heteroatoms, and each heteroatom is independently and optionally selected from nitrogen, oxygen, and sulfur (5- to 8-membered heterocycloalkyl). Heterocycloalkyl is a 5- to 6-membered non-aromatic ring containing ring carbon atoms and 1 to 4 ring heteroatoms, and each heteroatom is independently and optionally selected from nitrogen, oxygen, and sulfur (5- to 6-membered heterocycloalkyl). In some embodiments, 5- to 6-membered heterocycloalkyl contains 1 to 3 ring heteroatoms independently selected from nitrogen, oxygen, and sulfur. In some embodiments, 5- to 6-membered heterocycloalkyl contains 1 to 2 ring heteroatoms independently selected from nitrogen, oxygen, and sulfur. In some embodiments, 5- to 6-membered heterocycloalkyl contains 1 ring heteroatom independently selected from nitrogen, oxygen, and sulfur. Partially unsaturated heterocycloalkyl may be referred to as "heterocycloalkenyl" if heterocycloalkyl contains at least one double bond, or "heterocycloalkynyl" if the heterocycloalkyl contains at least one triple bond. Heterocycloalkyl may include monocyclic, bicyclic, spiro ring, or polycyclic (e.g., having two fused or bridged rings) systems. In some embodiments, heterocycloalkyl is a monocyclic group having 1, 2, or 3 heteroatoms independently selected from nitrogen, sulfur, and oxygen. The ring carbon atoms and heteroatoms of heterocycloalkyl may optionally be oxidized to form oxo or thio groups or other oxidized bonds (e.g., C(O), S(O), C(S) or S(O)₂, and N-oxides), or the nitrogen atoms may be quaternized. Heterocycloalkyl may be attached via a ring carbon atom or a ring heteroatom. In some embodiments, heterocycloalkyl contains 0 to 3 double bonds. In some embodiments, heterocycloalkyl contains 0 to 2 double bonds. The definition of heterocycloalkyl further includes moieties (also referred to as partially unsaturated heterocyclic rings) having one or more aromatic rings fused to (i.e., sharing a bond with) the heterocycloalkyl ring, for example, benzo-derivatives of piperidine, morpholine, azepin, and thienyl. Heterocycloalkyl containing a fused aromatic ring may be attached via any ring atom, including ring atoms of the fused aromatic ring. Examples of heterocycloalkyl include, but are not limited to, azetidinyl, azepinyl, dihydrobenzofuranyl, dihydrofuranyl, dihydropyranyl, N-morpholinyl, 3-oxa-9-azaspiro[5.5]undecyl, 1-oxa-8-azaspiro[4.5]decyl, piperidinyl, piperazinyl, oxopiperazinyl, pyranyl, pyrrolidinyl, quininyl, tetrahydrofuranyl, tetrahydropyranyl, 1,2,3,4-tetrahydroquinolinyl, tropanyl, 4,5,6,7-tetrahydrothiazolo[5,4-c]pyridinyl, 4,5,6,7-tetrahydro-1H-imidazo[4,5-c]pyridine, N-methylpiperidinyl, tetrahydroimidazolyl, pyrazolidinyl, butyrolactam, valerolactam, imidazolidinonyl, hydantoinyl, dioxolanyl, phthalimidyl, pyrimidine-2,4(1H,3H)-dione, 1,4-dioxanyl, morpholinyl, thiomorpholinyl, thiomorpholinyl-S-oxide, thiomorpholinyl-S,S-oxide, piperazinyl, pyranyl, pyridonyl, 3-pyrrolinyl, thiopyranyl, pyronyl, tetrahydrothienyl, 2-azaspiro[3.3]heptanyl, indolinyl,

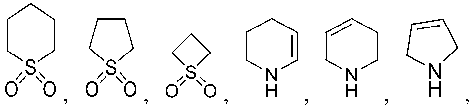

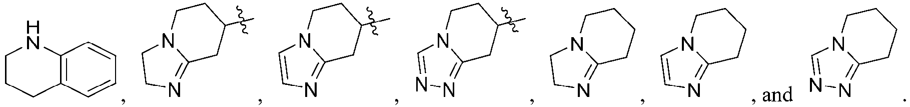

Unless otherwise specified, "heterocycloalkylene" refers to a divalent heterocycloalkyl as defined above.

Unless otherwise specified, "heterocyclic spirocycloalkyl" refers to a polycyclic hydrocarbyl formed by sharing one carbon atom (referred to as a spiro atom) between two or more saturated or partially unsaturated monocyclic rings, wherein one or more (e.g., 1, 2, or 3) ring atoms are heteroatoms selected from nitrogen, oxygen, and S(O)ₚ (where p is 0, 1, or 2), and the remaining ring atoms are carbon atoms. When the heteroatom is a nitrogen atom, the nitrogen atom may be substituted or unsubstituted (i.e., N or NR, R being hydrogen or other substituents already defined herein). Each monocyclic ring may contain one or more double bonds, but none of the rings has a fully conjugated π-electron system. According to the number of spiro atoms shared among the rings, the spiro heterocyclyl may be mono-spiro heterocyclyl, di-spiro heterocyclyl, or poly-spiro heterocyclyl. The term "(5-15 membered) heterocyclic spirocycloalkyl" refers to a heterocyclic spirocycloalkyl having 5 to 15 ring atoms, wherein the monocyclic ring that shares a spiro atom is a 3- to 8-membered monocyclic ring, and at least 1 monocyclic ring is a heterocycloalkyl ring. Preferred is (6-18 membered) heterocyclic spirocycloalkyl having 6 to 18 ring atoms, of which 1-3 ring atoms are heteroatoms. More preferred is (7-15 membered) heterocyclic spirocycloalkyl having 7 to 15 ring atoms, of which 1-3 ring atoms are heteroatoms. Most preferred is 9-membered (4-membered monocyclic (heterocyclyl) ring/6-membered monocyclic (heterocyclyl) ring, 5-membered monocyclic (heterocyclyl) ring/5-membered monocyclic (heterocyclyl) ring) mono-spiro heterocyclyl, 10-membered (5-membered monocyclic (heterocyclyl) ring/6-membered monocyclic (heterocyclyl) ring) mono-spiro heterocyclyl, or 11-membered (6-membered monocyclic (heterocyclyl) ring/6-membered monocyclic (heterocyclyl) ring) mono-spiro heterocyclyl. Specific examples of heterocyclic spirocycloalkyl include, but are not limited to,

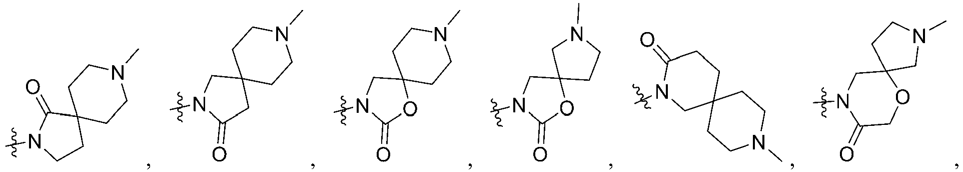

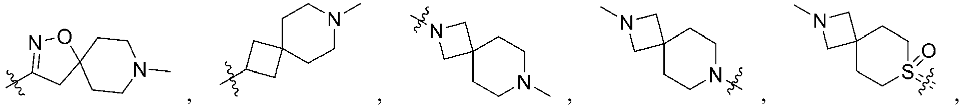

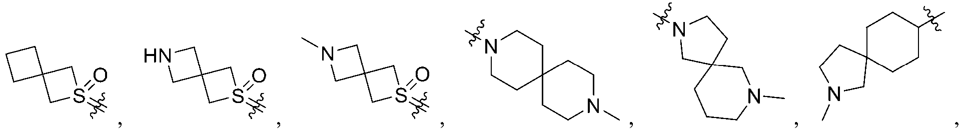

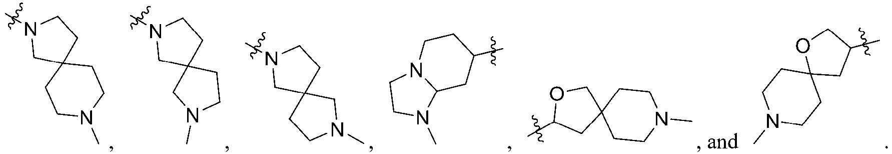

Unless otherwise specified, "oxo" refers to =O; for example, a group formed by substitution of carbon with one oxo is "carbonyl ( 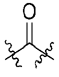 ) "; a group formed by substitution of sulfur with one oxo is "sulfinyl ( 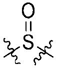 ) ", and a group formed by substitution of sulfur with two oxo is "sulfonyl ( 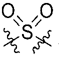 )".

Unless otherwise specified, "halogen" (or halo) refers to fluorine, chlorine, bromine, or iodine. The term "halo" (or "halogenated") before a group name indicates that the group is partially or fully halogenated, that is, substituted in any combination with F, Cl, Br, or I, preferably with F or Cl.

Unless otherwise specified, the term "substituted" means that one or more hydrogen atoms on a designated atom or group are substituted with one or more substituents other than hydrogen without exceeding the normal valence of the designated atom. For example, one or more hydrogens of alkyl, alkylene, alkenyl, alkynyl, hydroxy, amino, or the like may be substituted with one or more substituents. The substituents include, but are not limited to, alkyl, alkenyl, alkynyl, alkoxy, acyl, amino, amido, amidino, aryl, azido, carbamoyl, carboxy, carboxylate, cyano, guanidino, halogen, haloalkyl, heteroalkyl, heteroaryl, heterocyclyl, hydroxy, hydrazino, imino, oxo, nitro, alkylsulfinyl, sulfonic acid, alkylsulfonyl, thiocyanate, thiol, thione, or combinations thereof. The definition of "substituted" does not include analogous indeterminate structures obtained by defining substituents having further substituents attached to infinity (e.g., substituted aryl having substituted alkyl is itself substituted with substituted aryl, which is further substituted with substituted heteroalkyl, and the like). Unless otherwise specified, the maximum number of consecutive substitutions in the compound described herein is three. For example, the consecutive substitutions of substituted aryl with two other substituted aryls are limited to ((substituted aryl)substituted aryl)substituted aryl. Similarly, the definitions described above do not include impermissible substitution patterns (e.g., methyl substituted with 5 fluorines or heteroaryl having two adjacent oxygen ring atoms). Such impermissible substitution patterns are well known to those skilled in the art. When used to modify a chemical group, "substituted" may describe other chemical groups as defined herein. For example, the term "substituted aryl" includes, but is not limited to, "alkylaryl". Unless otherwise specified, if a group is described as optionally substituted, any substituent of the group is itself unsubstituted. "Optional" or "optionally" means that the subsequently described event or circumstance may, but does not necessarily, occur, and the description includes instances where the event or circumstance occurs and instances where it does not.

Unless otherwise specified, "acyl" refers to -C(=O)-R, wherein R is selected from optionally substituted alkyl, alkenyl, alkynyl, cycloalkyl, cycloalkenyl, cycloalkynyl, aryl, heteroaryl or heterocycloalkyl.

Unless otherwise specified, it will be understood that the word "comprise" or variations thereof such as "comprises" or "comprising" refers to the inclusion of a stated element or integer or a group of elements or integers, but not the exclusion of any other element or integer or a group of elements or integers.

The substituent "-O-CH₂-O-" means that two oxygen atoms in the substituent are attached to two adjacent carbon atoms in the heterocycloalkyl, aryl, or heteroaryl, for example:

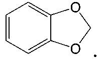

When the number of a linker group is 0, such as -(CH2)₀-, it means that the linker group is a single bond.

When one of the variables is selected from a chemical bond, it means that the two groups attached via this variable are linked directly. For example, when L in X-L-Y represents a chemical bond, it means that the structure is actually X-Y.

The term "membered ring" includes any cyclic structure. The term "membered" is intended to refer to the number of backbone atoms that form a ring. For example, cyclohexyl, pyridinyl, pyranyl, and thiopyranyl are six-membered rings, and cyclopentyl, pyrrolyl, furanyl, and thienyl are five-membered rings.

The term "moiety" refers to a specific portion or functional group of a molecule. A chemical moiety is generally considered to be a chemical entity contained in or attached to a molecule.

The term "isomer" refers to any tautomer, stereoisomer, atropisomer, isotopic isomer, enantiomer, or diastereomer of any compound of the present disclosure. The compound of the present disclosure may have one or more chiral centers or double bonds, and thus exists in the form of stereoisomers, e.g., double bond isomers (i.e., E/Z geometric isomers), or diastereomers (e.g., enantiomers (i.e., (+) or (-)) or cis/trans isomers). The compound of the present disclosure therefore encompasses all corresponding stereoisomers, i.e., stereoisomerically pure (e.g., geometrically pure, enantiomerically pure, or diastereomerically pure) forms, as well as mixtures of enantiomers and stereoisomers, e.g., racemates. The mixtures of enantiomers and stereoisomers of the compound of the present disclosure may be resolved into their component enantiomers or stereoisomers by well-known methods, such as chiral gas chromatography and chiral high-performance liquid chromatography, and by crystallization of the compound in the form of chiral salt complexes or in chiral solvents. Enantiomers and stereoisomers may also be obtained from stereomerically pure or enantiomerically pure intermediates, reagents, and catalysts by well-known asymmetric synthetic methods.

The term "isotopic isomer" refers to distinct molecules that differ in structure only by their isotopic composition and are identical in the remaining structure.

### Specific Pharmaceutical and Medical Terminology

The term "acceptable", as used herein, means that a formula component or an active ingredient does not unduly and adversely affect a general therapeutic target's health.

The terms "treatment", "treatment course", and "therapy", as used herein, include alleviating, inhibiting, or ameliorating a symptom or condition of a disease; inhibiting the development of complications; ameliorating or preventing underlying metabolic syndrome; inhibiting the development of a disease or symptom, e.g., controlling the progression of a disease or condition; alleviating a disease or symptom; leading to disease or symptom regression; and alleviating a complication caused by a disease or symptom, or preventing or treating a sign caused by a disease or symptom. As used herein, a compound or pharmaceutical composition, when administered, can ameliorate a disease, symptom, or condition, which particularly refers to ameliorating the severity, delaying the onset, slowing the progression, or reducing the duration of the disease. Fixed or temporary administration, or continuous or intermittent administration, may be attributed to or associated with the administration.

"Active ingredient" refers to the compound of general formula (1), and pharmaceutically acceptable inorganic or organic salts of the compound of general formula (1). The compound of the present disclosure may contain one or more asymmetric centers (chiral center or axial chirality) and thus occurs in the forms of a racemate, a racemic mixture, a single enantiomer, a diastereomeric compound, and a single diastereomer. Asymmetric centers that may be present depend on the nature of the various substituents on the molecule. Each of such asymmetric centers will independently produce two optical isomers, and all possible optical isomers, diastereomeric mixtures and pure or partially pure compounds are included within the scope of the present disclosure. The present disclosure is meant to include all such isomeric forms of these compounds.

The terms such as "compound", "composition", "agent", or "medicine or medicament" are used interchangeably herein and all refer to a compound or composition that, when administered to an individual (human or animal), is capable of inducing a desired pharmacological and/or physiological response by local and/or systemic action.

The term "administered, administering, or administration" refers herein to the direct administration of the compound or composition, or the administration of a prodrug, derivative, analog, or the like of the active compound.

Although the numerical ranges and parameters defining the broad scope of the present disclosure are approximations, the related numerical values set forth in the specific examples have been presented herein as precisely as possible. Any numerical value, however, inherently contains a standard deviation necessarily resulting from certain methods of testing. Herein, "about" generally means that the actual numerical value is within a particular numerical value or range ± 10%, 5%, 1%, or 0.5%. Alternatively, the term "about" indicates that the actual numerical value falls within the acceptable standard error of a mean, as considered by those skilled in the art. All ranges, quantities, numerical values, and percentages used herein (e.g., to describe an amount of a material, a length of time, a temperature, an operating condition, a quantitative ratio, and the like) are to be understood as being modified by the word "about", except in the experimental examples or where otherwise explicitly indicated. Accordingly, unless otherwise contrarily stated, the numerical parameters set forth in the specification and the appended claims are all approximations that may vary as desired. At least, these numerical parameters should be understood as the significant digits indicated or the numerical values obtained using conventional rounding rules.

Unless otherwise defined in the specification, the scientific and technical terms used herein have the same meaning as commonly understood by those skilled in the art. Furthermore, nouns in their singular forms used in the specification encompass their plural forms, unless contradicted by context; nouns in their plural forms used also encompass their singular forms.

### Therapeutic Use

The present disclosure provides a method for treating a disease, including but not limited to a related condition involving an HDAC enzyme (e.g., cancer), with the compound of general formula (1) or the pharmaceutical compositions of the present disclosure.

In some embodiments, a method for treating cancer is provided, the method including administering to an individual in need thereof an effective amount of any of the aforementioned pharmaceutical compositions comprising the compound of general formula (1). In some embodiments, the cancer is mediated by a related HDAC enzyme. In some embodiments, the compound of the present disclosure is used in combination with an immune checkpoint inhibitor; in some embodiments, the compound of the present disclosure is used in combination with a PD-1 or PD-L1 inhibitor; in some embodiments, the compound of the present disclosure is used in combination with a PD-1 antibody; in some embodiments, the compound of the present disclosure is used in combination with a PD-L1 antibody; in some embodiments, the compound of the present disclosure is used in combination with a VEGF/VEGFR inhibitor; in some embodiments, the compound of the present disclosure is used in combination with an immune checkpoint inhibitor and a VEGF/VEGFR inhibitor; in some embodiments, the compound of the present disclosure is used in combination with a PD-1 inhibitor and a VEGF/VEGFR inhibitor; in some embodiments, the compound of the present disclosure is used in combination with a PD-1 antibody and a VEGF/VEGFR inhibitor; the PD-1 antibody includes, but is not limited to, nivolumab, pembrolizumab, toripalimab, sintilimab, camrelizumab, tislelizumab, penpulimab, zimberelimab, serplulimab, pucotenlimab, pidilizumab, cemiplimab, spartalizumab, AMG404, RN888, mAbl5, MEDI-0680, BGB-108, spartalizumab, IBI-308, mDX-400, SHR-1210, PF-06801591, PDR-001, GB-226, and STI-1110, and biosimilars, biobetters, and bioequivalents of these inhibitors; the PD-L1 antibody includes, but is not limited to, durvalumab, atezolizumab, envafolimab, sugemalimab, avelumab, avelumab, BMS-936559, AMP-714, ALN-PDL, TSR-042, KD-033, CA-170, STI-1014, and KY-1003, and biosimilars, biobetters, and bioequivalents of these inhibitors; the VEGF/VEGFR inhibitor includes, but is not limited to, bevacizumab, ranibizumab, ramucirumab, sorafenib, axitinib, apatinib, sunitinib, regorafenib, vandetanib, pazopanib, lenvatinib, cabozantinib, ponatinib, aflibercept, and fruquintinib. In other embodiments, the tumor includes, but is not limited to: breast cancer, colon cancer, uterine cancer, pancreatic cancer, lung cancer, gastric cancer, leukemia, lymphoma, prostate cancer, liver cancer, cervical cancer, neuroblastoma, melanoma, or intracranial tumors.

### Route of Administration

The compound and the pharmaceutically acceptable salt thereof of the present disclosure can be made into various formulations comprising a safe and effective amount of the compound or the pharmaceutically acceptable salt thereof of the present disclosure, and a pharmaceutically acceptable excipient or carrier. The "safe and effective amount" means that the amount of the compound is sufficient to significantly improve the condition without causing serious side effects. The safe and effective amount of the compound is determined according to the age, condition, course of treatment, and other specific conditions of a treated subject.

The "pharmaceutically acceptable excipient or carrier" refers to one or more compatible solid or liquid fillers or gel substances that are suitable for human use and must be of sufficient purity and sufficiently low toxicity. "Compatible" herein means that the components of the composition are capable of intermixing with the compound of the present disclosure and with each other, without significantly diminishing the pharmaceutical efficacy of the compound. Examples of pharmaceutically acceptable excipients or carriers include cellulose and derivatives thereof (e.g., sodium carboxymethylcellulose, sodium ethylcellulose, or cellulose acetate), gelatin, talc, solid lubricants (e.g., stearic acid or magnesium stearate), calcium sulfate, vegetable oil (e.g., soybean oil, sesame oil, peanut oil, or olive oil), polyols (e.g., propylene glycol, glycerol, mannitol, or sorbitol), emulsifiers (e.g., Tween^{®}), wetting agents (e.g., sodium lauryl sulfate), colorants, flavoring agents, stabilizers, antioxidants, preservatives, pyrogen-free water, etc.

When the compound of the present disclosure is administered, it may be administered orally, rectally, parenterally (intravenously, intramuscularly, or subcutaneously), or topically.

Solid dosage forms for oral administration include capsules, tablets, pills, pulvises, and granules. In these solid dosage forms, the active compound is mixed with at least one conventional inert excipient (or carrier), such as sodium citrate or dicalcium phosphate, or with the following ingredients: (a) fillers or extenders, such as starch, lactose, sucrose, glucose, mannitol, and silicic acid; (b) binders, such as hydroxymethyl cellulose, alginate, gelatin, polyvinylpyrrolidone, sucrose, and acacia; (c) humectants, such as glycerol; (d) disintegrants, such as agar, calcium carbonate, potato or tapioca starch, alginic acid, certain complex silicates, and sodium carbonate; (e) solution retarders, such as paraffin; (f) absorption accelerators, such as quaternary ammonium compounds; (g) wetting agents, such as cetyl alcohol and glycerol monostearate; (h) adsorbents, such as kaolin; and (i) lubricants, such as talc, calcium stearate, magnesium stearate, solid polyethylene glycol and sodium lauryl sulfate, or mixtures thereof. In the case of capsules, tablets, and pills, the dosage forms may further include buffers.

Solid dosage forms such as tablets, dragees, capsules, pills, and granules can be prepared using coatings and shells such as enteric coatings and other materials well known in the art. They may include opacifying agents, and the active compound or compound in such a composition may be released in a certain part of the digestive tract in a delayed manner. Examples of embedding components that can be used are polymeric substances and wax-based substances. If necessary, the active compound can also be in microcapsule form with one or more of the excipients described above.

Liquid dosage forms for oral administration include pharmaceutically acceptable emulsions, solutions, suspensions, syrups, and elixirs. In addition to the active compound, the liquid dosage form may include inert diluents commonly used in the art, such as water or other solvents, solubilizers and emulsifiers, for example, ethanol, isopropanol, ethyl carbonate, ethyl acetate, propylene glycol, 1,3-butanediol, dimethylformamide, and oils, especially cottonseed oil, peanut oil, corn germ oil, olive oil, castor oil and sesame oil, or mixtures of these substances.

Besides such inert diluents, the composition may further include adjuvants, such as wetting agents, emulsifiers, suspending agents, sweeteners, flavoring agents, and perfuming agents.

In addition to the active compound, suspensions may include suspending agents, such as ethoxylated isostearyl alcohols, polyoxyethylene sorbitol and sorbitan esters, microcrystalline cellulose, aluminum methylate and agar, or mixtures of these substances.

Compositions for parenteral injection may include physiologically acceptable sterile aqueous or anhydrous solutions, dispersions, suspensions, or emulsions, and sterile powders for redissolving into sterile injectable solutions or dispersions. Suitable aqueous and non-aqueous carriers, diluents, solvents, or excipients include water, ethanol, polyols, and suitable mixtures thereof.

Dosage forms for topical administration of the compound of the present disclosure include ointments, pulvises, patches, sprays, and inhalants. The active ingredient is mixed under sterile conditions with a physiologically acceptable carrier and any preservatives, buffers, or propellants that may be required if necessary.

The compound of the present disclosure may be administered alone or in combination with other pharmaceutically acceptable compounds. When the pharmaceutical composition is used, a safe and effective amount of the compound of the present disclosure is administered to a mammal (such as a human) to be treated, wherein the dose of administration is a pharmaceutically effective dose. For a human of 60 kg, the daily dose of administration is usually 1-2000 mg, preferably 50-1000 mg. In determining a specific dose, such factors as the route of administration, the health condition of the patient, and the like will also be considered, which are well-known to skilled physicians.

The above features mentioned in the present disclosure or those mentioned in the examples may be combined arbitrarily. All the features disclosed in this specification may be used with any composition form and the various features disclosed in this specification may be replaced with any alternative features that provide the same, equivalent, or similar purpose. Thus, unless otherwise specified, the features disclosed herein are merely general examples of equivalent or similar features.

### DETAILED DESCRIPTION

Various specific aspects, features, and advantages of the compounds, methods, and pharmaceutical compositions described above will be set forth in detail in the following description, which will make the content of the present disclosure very clear. It should be understood that the detailed description and examples below describe specific examples for reference only. After reading the description of the present disclosure, those skilled in the art can make various changes or modifications to the present disclosure, and such equivalents also fall within the scope of the present application defined herein.

In all the examples, ¹H-NMR spectra were recorded with a Varian Mercury 400 nuclear magnetic resonance spectrometer, and chemical shifts are represented by δ (ppm); silica gel for separation was 200-300 mesh silica gel if not specified, and the ratio of the eluents was a volume ratio.

The following abbreviations are used in the present disclosure: AcOH represents glacial acetic acid; Boc₂O represents di-tert-butyl dicarbonate; CDCl₃ represents deuterated chloroform; CD₃I represents deuterated iodomethane; Cs₂CO₃ represents cesium carbonate; DCM represents dichloromethane; DCE represents 1,2-dichloroethane; Diox represents 1,4-dioxane; DIPEA represents diisopropylethylamine; DMSO represents dimethyl sulfoxide; DMAP represents 4-dimethylaminopyridine; DMF represents N,N-dimethylformamide; EA represents ethyl acetate; EtOH represents absolute ethanol; Flash represents flash preparative mediumpressure liquid chromatography; h represents hour; H₂ represents hydrogen; HATU represents O-(7-azabenzotriazol-1-yl)-N,N,N',N'-tetramethyluronium hexafluorophosphate; HCl represents hydrochloric acid; K₂CO₃ represents anhydrous potassium carbonate; K₃PO₄ represents anhydrous potassium phosphate; LC-MS represents liquid chromatography-mass spectrometry; LiOH represents lithium hydroxide; LiOH.H₂O represents lithium hydroxide monohydrate; MeOH represents anhydrous methanol; min represents minute; mL represents milliliter; LC-MS represents mass spectrometry; NaBH(OAc)₃ represents sodium triacetoxyborohydride; NMR represents nuclear magnetic resonance; NH₄OAc represents ammonium acetate; Pd/C represents palladium on carbon; Pd(dppf)₂Cl₂ represents [1,1'-bis(diphenylphosphino)ferrocene]palladium dichloride dichloromethane complex; Pd(OAc)₂ represents palladium acetate; TFA represents trifluoroacetic acid; THF represents tetrahydrofuran; Xantphos represents 4,5-bis(diphenylphosphino)-9,9-dimethylxanthene; Zn represents zinc powder.

### Preparation Example 1. Synthesis of 4-((Dimethylsulfinylidene)amino)benzoic acid (S1-1)

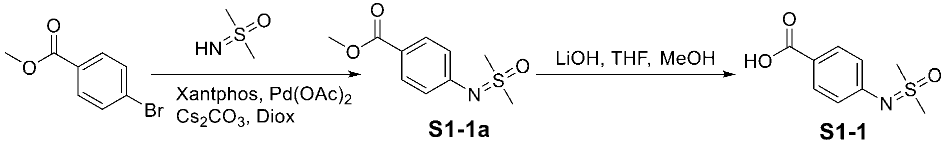

### Synthesis of S1-1a:

Methyl 4-bromobenzoate (21.5 g, 100.0 mmol), Xantphos (5.78 g, 10 mmol), Cs₂CO₃ (81.5 g, 250.0 mmol), Diox (500 mL), and dimethylsulfoximine (10.23 g, 110.0 mmol) were added to a 1 L single-neck flask. After the mixed solution was purged with argon, Pd(OAc)₂ (1.13 g, 5.0 mmol) was added. The mixed solution was heated to 100 °C and stirred for 8 h under argon atmosphere. After the completion of the reaction as detected by LC-MS, the mixture was cooled and filtered. The filter cake was rinsed with EA (about 100 mL). The filtrate was concentrated. Water (200 mL) and EA (200 mL) were added to the residue. The resulting mixture was stirred, and liquid separation was performed. The organic phase was then washed with a saturated sodium chloride solution and concentrated. The residue was purified by column chromatography to give a pale brown oily product (19.3 g, yield: 85%).

ESI-MS m/z: 228.1 [M+H]⁺.

### Synthesis of S1-1:

The above compound **S1-1a** (19.3 g, 85.02 mmol), THF (300 mL), MeOH (100 mL), water (100 mL), and lithium hydroxide monohydrate (14.28 g, 340.0 mmol) were added to a 1 L single-neck flask, and the mixed solution was stirred at room temperature for 20 h. After the completion of the reaction as detected by LC-MS, the mixed solution was concentrated until there was about one-third of the volume left, and the pH was then adjusted to 2-3 with 2 N HCl. The mixture was extracted twice with EA (200 mL × 2). The organic phases were combined, washed with a saturated sodium chloride solution, dried over anhydrous sodium sulfate, filtered, and concentrated to give an off-white solid product (15.2 g, yield: 83.9%).

ESI-MS m/z: 214.1 [M+H]⁺.

### Preparation Example 2. Synthesis of 4-((4-Methyl-1-oxo-1^{λ}6-thiomorpholin-1-imine)amino)benzoic acid (S1-2)

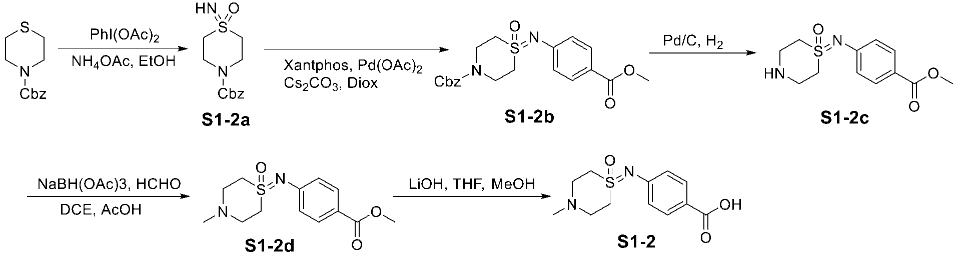

### Synthesis of S1-2a:

N-Boc-thiomorpholine (1 g, 4.22 mmol), EtOH (20 mL), ammonium acetate (1.3 g, 16.88 mmol), and iodobenzene diacetate (4.07 g, 12.66 mmol) were added to a 100 mL single-neck flask. The mixed solution was purged with argon and then stirred at room temperature for 20 h. After the completion of the reaction as detected by LC-MS, the mixed solution was concentrated. Water (50 mL) and EA (50 mL) were added to the residue. The resulting mixture was stirred, and liquid separation was performed. The organic phase was then washed with a saturated sodium chloride solution and concentrated. The residue was purified by column chromatography to give a pale brown oily product (925 mg, yield: 81.8%).

ESI-MS m/z: 269.1 [M+H]⁺.

### Synthesis of S1-2b:

Methyl 4-bromobenzoate (741 mg, 3.45 mmol), Xantphos (279 mg, 0.5 mmol), Cs₂CO₃ (2.81 g, 8.62 mmol), Diox (20 mL), and **S1-2a** (925 mg, 3.45 mmol) were added to a 1 L single-neck flask. After the mixed solution was purged with argon, Pd(OAc)₂ (113 mg, 0.5 mmol) was added. The mixed solution was heated to 100 °C and stirred for 4 h under argon atmosphere. After the completion of the reaction as detected by LC-MS, the mixture was cooled and filtered. The filter cake was rinsed with EA (about 20 mL), and the filtrate was concentrated. Water (30 mL) and EA (30 mL) were added to the residue. The resulting mixture was stirred, and liquid separation was performed. The organic phase was then washed with a saturated sodium chloride solution and concentrated. The residue was purified by column chromatography to give a pale brown oily product (1.05 g, yield: 75.6%).

ESI-MS m/z: 403.1 [M+H]⁺.

### Synthesis of S1-2c:

The above compound **S1-2b** (900 mg, 2.24 mmol), MeOH (10 mL), and 10% Pd/C (100 mg) were added to a 250 mL single-neck flask. The mixed solution was purged three times with hydrogen and then stirred at room temperature under normal pressure for 20 h. After the completion of the reaction as detected by LC-MS, the mixture was filtered through diatomite, and the filtrate was concentrated to dryness to give an off-white solid product (624 mg, yield: 100%).

ESI-MS m/z: 269.1 [M+H]⁺.

### Synthesis of S1-2d:

The above compound **S1-2c** (104 mg, crude, 0.373 mmol), DCE (5 mL), AcOH (30 mg, 0.5 mmol), and paraformaldehyde (16 mg, 0.533 mmol) were added to a 50 mL single-neck flask. The mixed solution was stirred at room temperature for 30 min, and then NaBH(OAc)₃ (158 mg, 0.746 mmol) was added. The mixed solution was stirred at room temperature for 20 h. After the completion of the reaction as detected by LC-MS, DCM (10 mL) and water (10 mL) were added to the system. The resulting mixture was stirred, liquid separation was performed, and the aqueous phase was then extracted with DCM (10 mL). The organic phases were combined, dried over anhydrous sodium sulfate, filtered, and concentrated to obtain a crude product.

ESI-MS m/z: 283.1 [M+H]⁺.

### Synthesis of S1-2:

The above compound **S1-2d** (126 mg, crude, 0.373 mmol), THF (5 mL), MeOH (2 mL), water (2 mL), and lithium hydroxide monohydrate (155 mg, 3.7 mmol) were added to a 50 mL single-neck flask. The mixed solution was stirred at room temperature for 2 h. After the completion of the reaction as detected by LC-MS, the mixed solution was concentrated, and the residue was purified by Flash and lyophilized to give an off-white solid product (76 mg, yield: 75.9%).

ESI-MS m/z: 269.1 [M+H]⁺.

### Preparation Example 3. Synthesis of 4-((4-Methyl-d3-1-oxo-1^{λ}6-thiomorpholin-1-imine)amino)benzoic acid (S1-3)

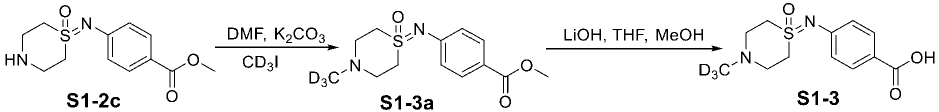

### Synthesis of S1-3a:

The above compound **S1-2c** (104 mg, crude, 0.373 mmol), DMF (5 mL), K₂CO₃ (155 mg, 1.12 mmol), and CD₃I (60 mg, 0.448 mmol) were added to a 25 mL single-neck flask. The mixed solution was stirred at room temperature for 2 h. After the completion of the reaction as detected by LC-MS, EA (10 mL) and water (10 mL) were added to the system. The resulting mixture was stirred, liquid separation was performed, and the aqueous phase was then extracted with EA (10 mL). The organic phases were combined, dried over anhydrous sodium sulfate, filtered, and concentrated to obtain a crude product (156 mg).

ESI-MS m/z: 286.1 [M+H]⁺.

### Synthesis of S1-3:

The above compound **S1-3a** (156 mg, crude, 0.373 mmol), THF (5 mL), MeOH (2 mL), water (2 mL), and lithium hydroxide monohydrate (155 mg, 3.7 mmol) were added to a 25 mL single-neck flask. The mixed solution was stirred at room temperature for 2 h. After the completion of the reaction as detected by LC-MS, the mixed solution was concentrated, and the residue was purified by Flash and lyophilized to give an off-white solid product (57 mg, yield: 56.3%).

ESI-MS m/z: 272.1 [M+H]⁺.

Target intermediates S1-4 to S1-23 in Table 1 were obtained by using the synthesis methods of intermediate S1-1, S1-2 and S1-3 with different starting materials.

**Tablet. Intermediates S1-4 to S1-23**

| **Interm ediates** | **Structure** | **MS [M+H]⁺** | **Intermed iates** | **Structure** | **MS [M+H]⁺** |
|---|---|---|---|---|---|
| **S1-4** | 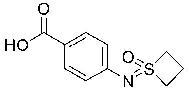 | 226.1 | **S1-5** | 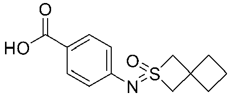 | 266.1 |
| **S1-6** | 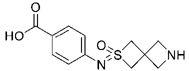 | 267.1 | **S1-7** | 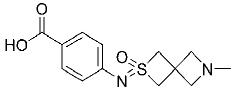 | 281.1 |
| **S1-8** | 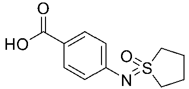 | 240.1 | **S1-9** | 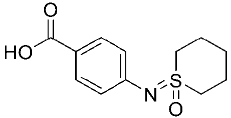 | 254.1 |
| **S1-10** | 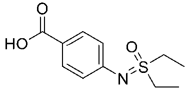 | 242.1 | **S1-11** | 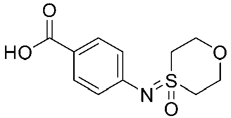 | 256.1 |
| **S1-12** | 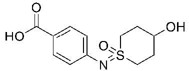 | 270.1 | **S1-13** | 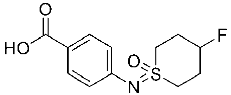 | 272.1 |
| **S1-14** | 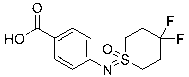 | 290.1 | **S1-15** | 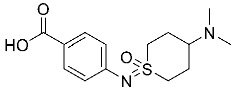 | 297.1 |
| **S1-16** | 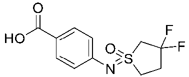 | 276.1 | **S1-17** | 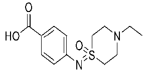 | 283.1 |
| **S1-18** | 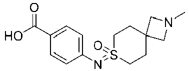 | 309.1 | **S1-19** | 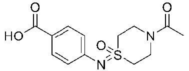 | 297.1 |
| **S1-20** | 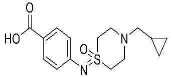 | 309.1 | **S1-21** | 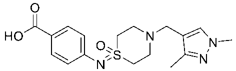 | 363.1 |
| **S1-22** | 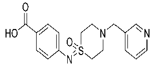 | 272.1 | **S1-23** | 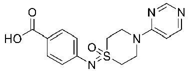 | 333.1 |

### Example 1. Synthesis of N-(4-Amino-4'-fluoro-[1,1'-biphenyl]-3-yl)-4-((dimethyl(oxo)-1^{λ}-6-sulfaneylidene)amino)benzamide (Compound 1)

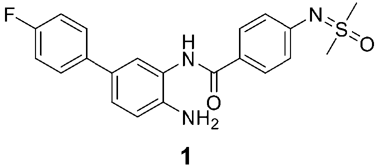

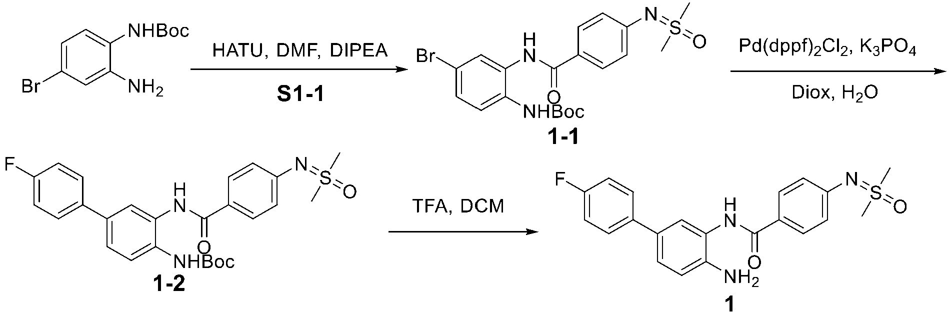

### Step 1: Synthesis of compound 1-1:

tert-butyl (2-Amino-4-bromophenyl)carbamate (5.0 g, 17.42 mmol), DIPEA (3.37 g, 26.13 mmol), S1-1 (3.71 g, 17.42 mmol), HATU (9.92 g, 26.13 mmol), and DMF (100 mL) were added to a 500 mL single-neck flask. The mixed solution was purged with argon and then stirred at room temperature for 20 h. After the completion of the reaction as detected by LC-MS, water (200 mL) was added to the mixed solution to quench the reaction, and the resulting mixture was extracted twice with EA (100 mL × 2). The organic phases were combined, washed with a saturated sodium chloride solution, dried over anhydrous sodium sulfate, filtered, and concentrated. The residue was purified by column chromatography to give a white solid product (7.22 g, yield: 86.0%).

ESI-MS m/z: 482.1 [M+H]⁺.

### Step 2: Synthesis of compound 1-2:

The above compound **1-1** (100 mg, 0.207 mmol), K₃PO₄ (110 mg, 0.52 mmol), Diox/H₂O (5 mL/1 mL), 4-fluorophenylboronic acid (35 mg, 0.25 mmol), and Pd(dppf)₂Cl₂ (14 mg) were added to a 100 mL single-neck flask. The mixed solution was purged with argon, heated to 100 °C, and stirred for 2 h. After the completion of the reaction as detected by LC-MS, the mixed solution was concentrated. The residue was purified by Flash to give a pale brown oily product (56 mg, yield: 54.4%).

ESI-MS m/z: 498.2 [M+H]⁺.

### Step 3: Synthesis of compound 1:

The above compound **1-2** (56 mg, 0.113 mmol), DCM (5 mL), and TFA (0.5 mL) were added to a 25 mL single-neck flask. The mixed solution was stirred at room temperature for 2 h. After the completion of the reaction as detected by LC-MS, the system was concentrated, and the residue was purified by Flash and lyophilized to give an off-white solid product (33 mg, yield: 73.5%).

ESI-MS m/z: 398.1 [M+H]⁺.

Tareget compounds **2-139** in Table 2 were obtained by using the synthesis methods of Compound 1 with different intermediates.

**Table 2**

| **Com poun d** | **Compound structure** | **MS (M+H)⁺** | **Com poun d** | **Compound structure** | **MS (M+H) +** |
|---|---|---|---|---|---|
| **2** | 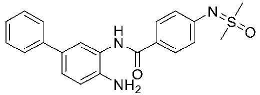 | 380.1 | **3** | 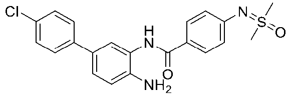 | 414.1 |
| **4** | 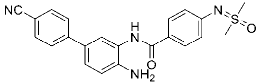 | 405.1 | **5** | 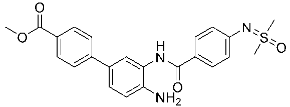 | 438.1 |
| **6** | 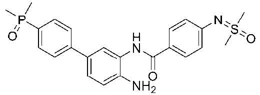 | 456.1 | **7** | 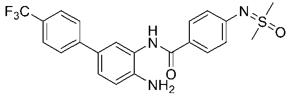 | 448.1 |
| **8** | 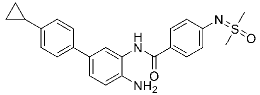 | 420.2 | **9** | 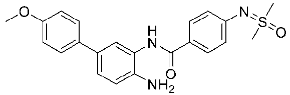 | 410.2 |
| **10** | 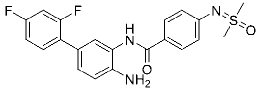 | 416.2 | **11** | 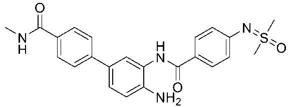 | 437.2 |
| **12** | 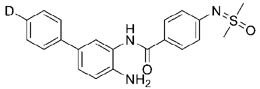 | 381.1 | **13** | 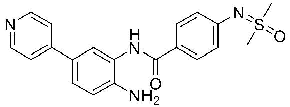 | 381.1 |
| **14** | 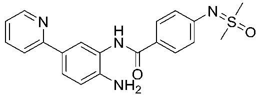 | 381.1 | **15** | 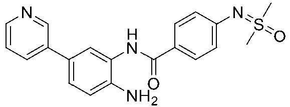 | 381.1 |
| **16** | 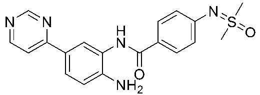 | 382.1 | **17** | 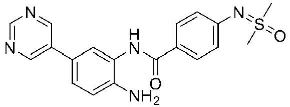 | 382.1 |
| **18** | 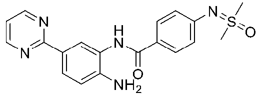 | 382.1 | **19** | 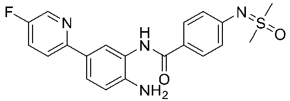 | 399.1 |
| **20** | 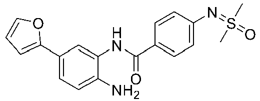 | 370.1 | **21** | 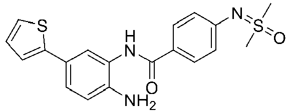 | 386.1 |
| **22** | 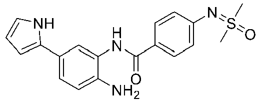 | 369.1 | **23** | 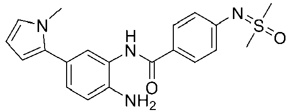 | 383.2 |
| **24** | 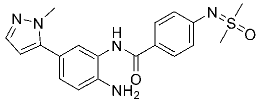 | 384.1 | **25** | 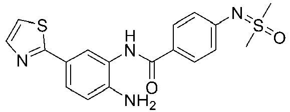 | 387.1 |
| **26** | 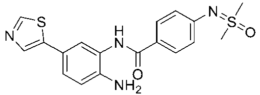 | 387.1 | **27** | 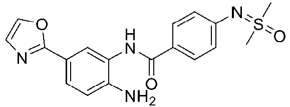 | 371.1 |
| **28** | 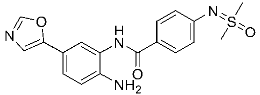 | 371.1 | **29** | 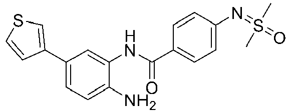 | 386.1 |
| **30** | 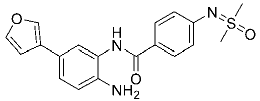 | 370.1 | **31** | 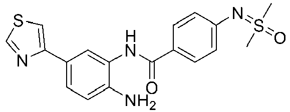 | 387.1 |
| **32** | 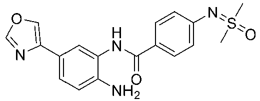 | 371.1 | **33** | 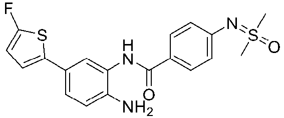 | 404.1 |
| **34** | 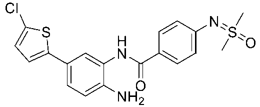 | 420.1 | **35** | 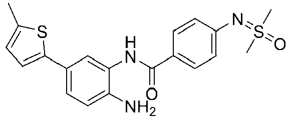 | 400.1 |
| **36** | 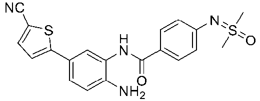 | 411.1 | **37** | 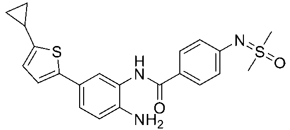 | 426.1 |
| **38** | 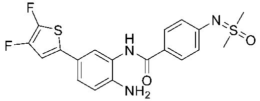 | 422.1 | **39** | 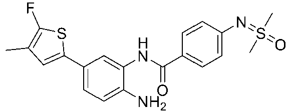 | 418.1 |
| **40** | 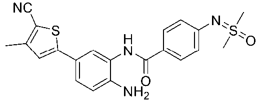 | 425.1 | **41** | 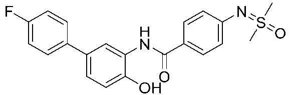 | 399.1 |
| **42** | 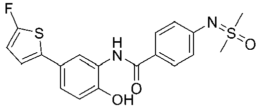 | 405.1 | **43** | 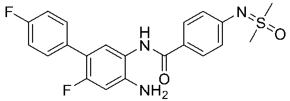 | 416.1 |
| **44** | 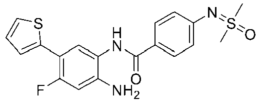 | 404.1 | **45** | 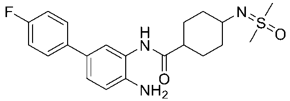 | 404.2 |
| **46** | 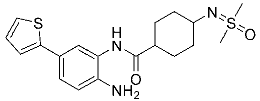 | 392.1 | **47** | 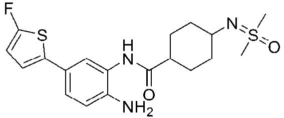 | 410.1 |
| **48** | 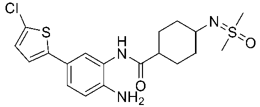 | 426.1 | **49** | 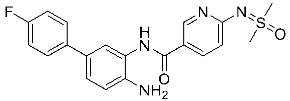 | 399.1 |
| **50** | 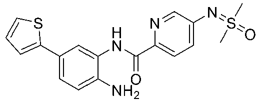 | 387.1 | **51** | 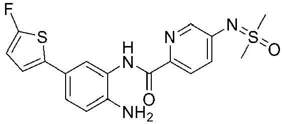 | 405.1 |
| **52** | 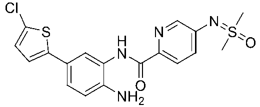 | 421.1 | **53** | 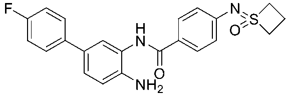 | 410.1 |
| **54** | 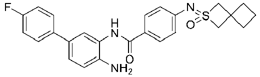 | 450.2 | **55** | 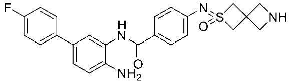 | 451.2 |
| **56** | 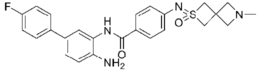 | 465.2 | **57** | 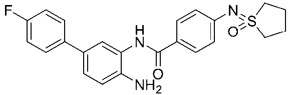 | 424.1 |
| **58** | 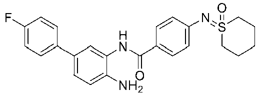 | 438.2 | **59** | 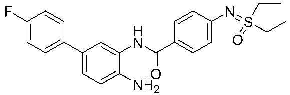 | 426.2 |
| **60** | 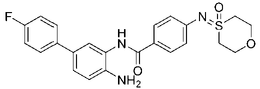 | 440.1 | **61** | 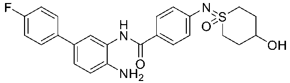 | 454.2 |
| **62** | 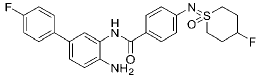 | 456.2 | **63** | 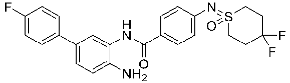 | 474.1 |
| **64** | 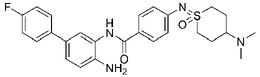 | 481.2 | **65** | 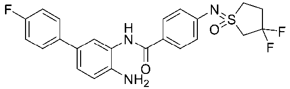 | 460.1 |
| **66** | 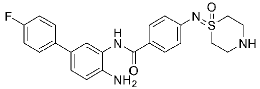 | 439.2 | **67** | 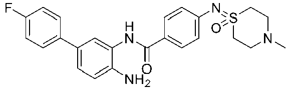 | 453.2 |
| **68** | 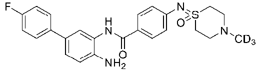 | 456.2 | **69** | 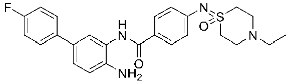 | 467.2 |
| **70** | 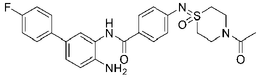 | 481.2 | **71** | 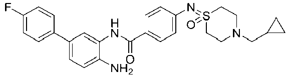 | 493.2 |
| **72** | 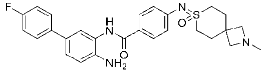 | 493.2 | **73** | 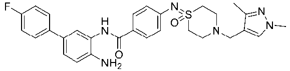 | 547.2 |
| **74** | 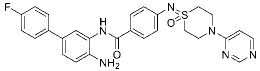 | 517.2 | **75** | 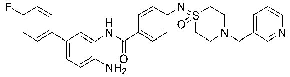 | 530.2 |
| **76** | 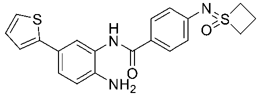 | 398.1 | **77** | 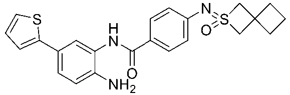 | 438.1 |
| **78** | 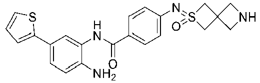 | 439.1 | **79** | 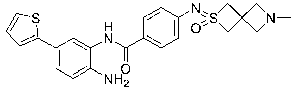 | 453.1 |
| **80** | 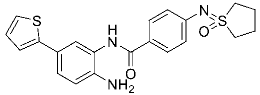 | 412.1 | **81** | 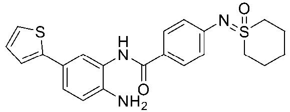 | 426.1 |
| **82** | 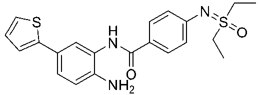 | 414.1 | **83** | 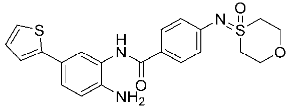 | 428.1 |
| **84** | 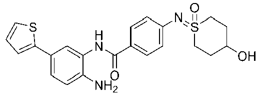 | 442.1 | **85** | 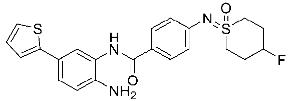 | 444.1 |
| **86** | 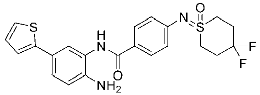 | 462.1 | **87** | 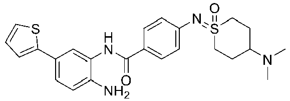 | 469.2 |
| **88** | 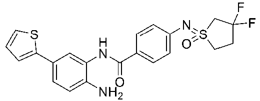 | 448.1 | **89** | 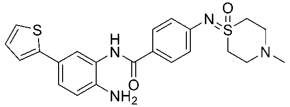 | 441.1 |
| **90** | 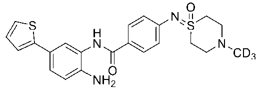 | 444.2 | **91** | 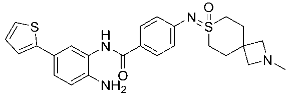 | 481.2 |
| **92** | 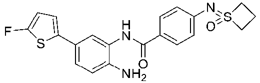 | 416.1 | **93** | 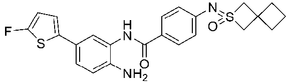 | 456.1 |
| **94** | 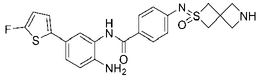 | 457.1 | **95** | 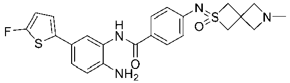 | 471.1 |
| **96** | 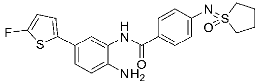 | 430.1 | **97** | 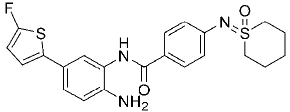 | 444.1 |
| **98** | 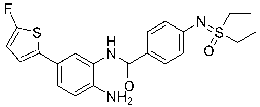 | 432.2 | **99** | 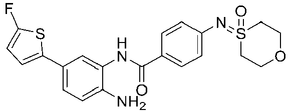 | 446.1 |
| **100** | 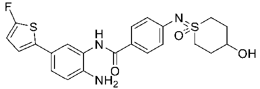 | 460.1 | **101** | 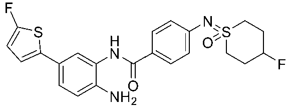 | 462.1 |
| **102** | 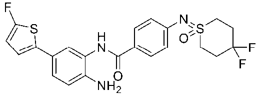 | 480.1 | **103** | 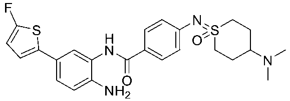 | 487.2 |
| **104** | 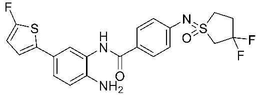 | 466.1 | **105** | 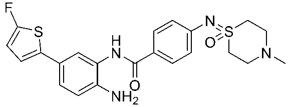 | 459.1 |
| **106** | 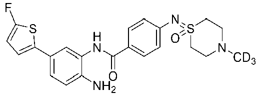 | 462.1 | **107** | 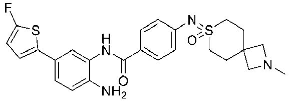 | 499.2 |
| **108** | 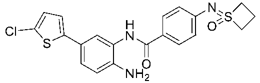 | 432.1 | **109** | 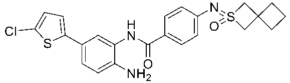 | 472.1 |
| **110** | 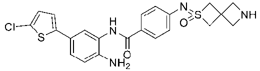 | 473.2 | **111** | 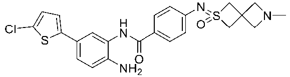 | 487.1 |
| **112** | 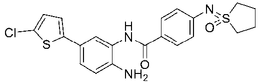 | 446.1 | **113** | 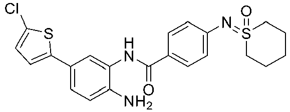 | 460.1 |
| **114** | 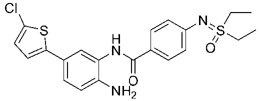 | 448.1 | **115** | 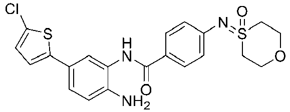 | 462.1 |
| **116** | 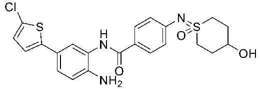 | 476.1 | **117** | 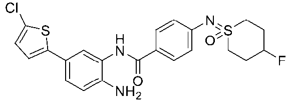 | 478.1 |
| **118** | 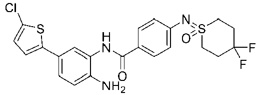 | 496.1 | **119** | 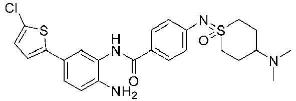 | 503.1 |
| **120** | 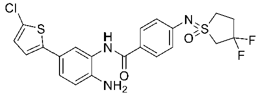 | 482.1 | **121** | 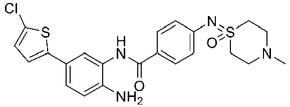 | 475.1 |
| **122** | 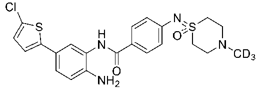 | 478.1 | **123** | 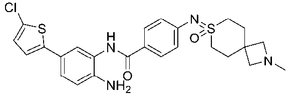 | 515.1 |
| **124** | 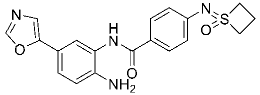 | 383.1 | **125** | 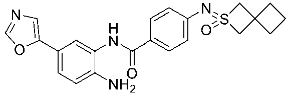 | 423.1 |
| **126** | 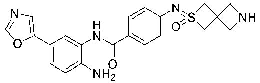 | 424.1 | **127** | 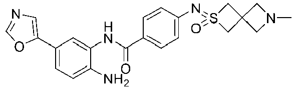 | 438.2 |
| **128** | 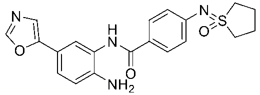 | 397.1 | **129** | 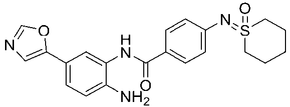 | 411.1 |
| **130** | 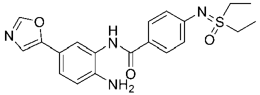 | 399.1 | **131** | 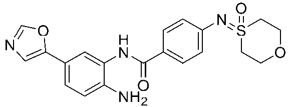 | 413.1 |
| **132** | 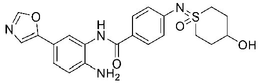 | 427.1 | **133** | 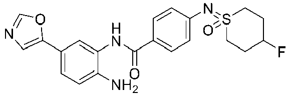 | 429.1 |
| **134** | 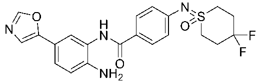 | 447.1 | **135** | 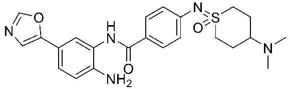 | 454.2 |
| **136** | 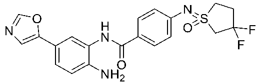 | 433.1 | **137** | 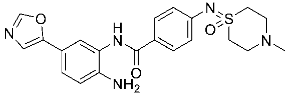 | 426.2 |
| **138** | 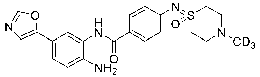 | 429.2 | **139** | 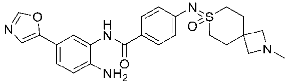 | 466.2 |

### Nuclear magnetic resonance data of some of the compounds of the present disclosure are listed in Table 3:

**Table 3**

| **Compoun d** | ¹ H NMR |
|---|---|
| **1** | ¹H NMR (400 MHz, DMSO-*d*₆) δ 9.52 (s, 1H), 7.85 (d, J = 8.6 Hz, 2H), 7.60 - 7.52 (m, 2H), 7.46 (d, J = 2.2 Hz, 1H), 7.27 (dd, J = 8.3, 2.3 Hz, 1H), 7.23 - 7.16 (m, 2H), 7.03 - 6.97 (m, 2H), 6.84 (d, J = 8.3 Hz, 1H), 5.04 (s, 2H), 3.27 (s, 6H). |
| **3** | ¹H NMR (400 MHz, DMSO-*d*₆) δ 9.52 (s, 1H), 7.85 (d, *J* = 8.3 Hz, 2H), 7.56 (d, *J* = 8.3 Hz, 2H), 7.50 (d, *J =* 2.2 Hz, 1H), 7.41 (d, *J =* 8.3 Hz, 2H), 7.30 (dd, *J =* 8.4, 2.2 Hz, 1H), 6.99 *(*d*, J =* 8.2 Hz, 2H), 6.84 (d, *J =* 8.3 Hz, 1H), 5.09 (s, 2H), 3.27 (s, 6H). |
| **33** | ¹H NMR (400 MHz, DMSO-*d*₆) δ 9.50 (s, 1H), 7.84 (d, *J* = 8.6 Hz, 2H), 7.35 (d, *J* = 2.2 Hz, 1H), 7.18 (dd, *J* = 8.3, 2.3 Hz, 1H), 6.99 (d, *J* = 8.6 Hz, 2H), 6.88 (t, *J* = 3.8 Hz, 1H), 6.78 (d, *J* = 8.4 Hz, 1H), 6.64 (dd, *J* = 4.1, 2.3 Hz, 1H), 5.11 (s, 2H), 3.27 (s, 6H). |
| **57** | ¹H NMR (400 MHz, DMSO-*d*₆) δ 10.02 (s, 1H), 7.94 (d, *J =* 8.2 Hz, 2H), 7.72 - 7.56 (m, 3H), 7.45 (d, *J =* 8.3 Hz, 1H), 7.32 - 7.13 (m, 3H), 6.99 (d, *J* = 8.2 Hz, 2H), 3.36 (p, *J* = 7.5, 7.0 Hz, 4H), 2.29 - 2.17 (m, 2H), 2.12 (dd, *J* = 11.8, 5.8 Hz, 2H). |

### Biological Example 1. Assay for Inhibitory Activity of Compounds of the Present Disclosure Against Various Subtypes of HDAC Enzymes

The compounds of the present disclosure were assayed for enzyme inhibitory activity against various subtypes of HDAC enzymes by the FRET method. The various subtypes of HDAC enzymes were obtained either through purification or direct purchase of assay kits.

The specific method was as follows: The enzyme was added to a 384-well plate, and a reaction buffer was added to the control well. Serially diluted DMSO sample solutions were added to the reaction wells, and the plate was incubated at room temperature for 15 min. Then a chromogenic agent was added to stop the reaction, and fluorescence was generated. The fluorescence intensity was measured using an EnVision multi-label microplate reader (excitation: 355 nM, emission: 460 nM). The endpoint value was read after the color development reached stability. The inhibition IC₅₀ of the compounds was calculated using GraphPad Prism4 software relative to DMSO.

**Table 4. Inhibitory activity of compound of the present disclosure against HDAC1, HDAC2, and HDAC3 (IC₅₀, nM)**

| **Compound** | **HDAC1** | **HDAC2** | **HDAC3** | **Compound** | **HDAC1** | **HDAC2** | **HDAC3** |
|---|---|---|---|---|---|---|---|
| **1** | A | A | C | **2** | A | A | C |
| **3** | A | A | C | **4** | A | A | C |
| **5** | A | B | C | **6** | B | B | C |
| **7** | A | A | C | **8** | B | B | C |
| **9** | A | A | C | **10** | A | A | C |
| **11** | A | A | C | **12** | A | A | C |
| **13** | A | A | C | **14** | A | A | C |
| **15** | A | A | C | **16** | A | A | C |
| **17** | A | A | C | **18** | A | A | C |
| **19** | A | A | C | **20** | A | A | C |
| **41** | A | B | C | **42** | A | A | C |
| **43** | A | A | C | **44** | A | A | C |
| **45** | A | A | C | **46** | A | A | C |
| **47** | A | A | C | **48** | A | A | C |
| **67** | A | A | C | **68** | A | A | C |
| **71** | A | A | C | **72** | A | A | C |
| **73** | A | A | C | **79** | A | A | C |
| **80** | A | A | C | **89** | A | A | C |
| **90** | A | A | C | **91** | A | A | C |
| **92** | A | A | C | **93** | A | A | C |
| **94** | A | A | C | **95** | A | A | C |
| **96** | A | A | C | | | | |

| | | | | | | | |
|---|---|---|---|---|---|---|---|
| A means that IC₅₀ is less than 500 nM; B means that IC ₅₀ is 500 nM to 5 uM; C means that IC₅₀ is greater than 5 uM | | | | | | | |

**Table 5. Inhibitory activity of compound of the present disclosure against HDAC1, HDAC2, and HDAC3 (IC₅₀, nM)**

| **Compound** | **HDAC1** | **HDAC2** | **HDAC3** | **Compound** | **HDAC1** | **HDAC2** | **HDAC3** |
|---|---|---|---|---|---|---|---|
| **1** | ++++ | +++ | + | **3** | ++++ | +++ | + |
| **33** | ++++ | +++ | + | **53** | ++++ | +++ | + |
| **54** | ++++ | +++ | + | **55** | ++++ | +++ | + |
| **56** | ++++ | +++ | + | **57** | ++++ | +++ | + |
| **58** | ++++ | +++ | + | **59** | ++++ | +++ | + |
| **60** | ++++ | +++ | + | **63** | ++++ | +++ | + |
| **65** | ++++ | +++ | + | **67** | ++++ | +++ | + |
| **68** | ++++ | +++ | + | **69** | ++++ | +++ | + |
| **72** | ++++ | +++ | + | **73** | ++++ | +++ | + |
| **Reference compounds** | +++ | +++ | + | | | | |

| | | | | | | | |
|---|---|---|---|---|---|---|---|
| +++ means that IC₅₀ ≤30nM; +++ means that30nM<IC₅₀≤300nM; ++ means that 300nM<IC₅₀≤3uM; + means that IC₅₀>3uM | | | | | | | |

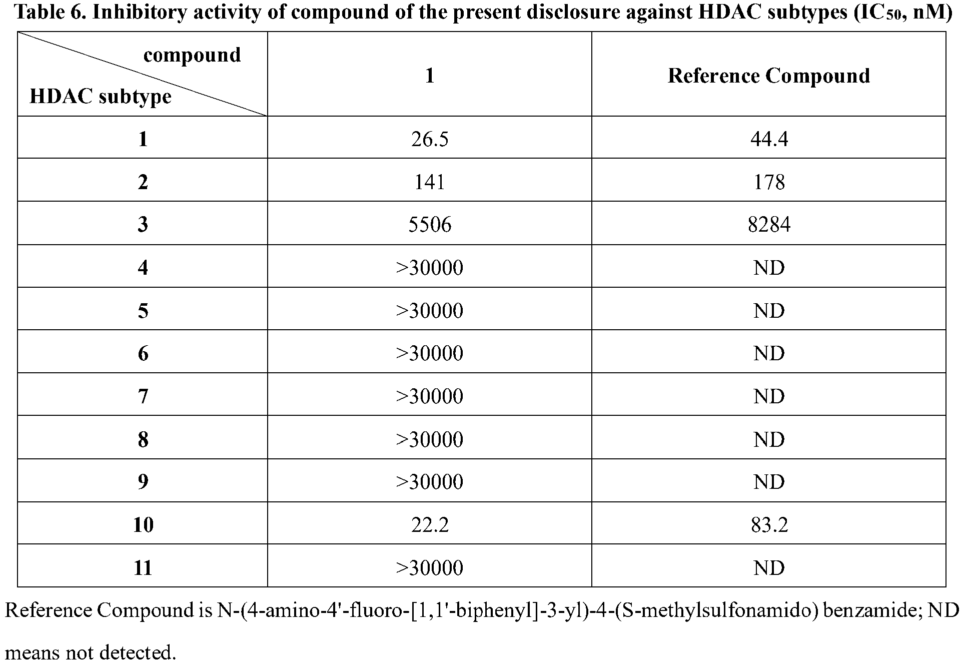

### Biological Example 2. Assay for Inhibition of Jurkat and 293T Cell Proliferation by Compounds of the Present Disclosure

Jurkat or 293T cells were seeded into a 96-well plate at 3000 cells/well. After overnight adherence culture, the serially diluted compound in DMSO was added. After 72 h, the intracellular ATP level was determined by CTG. The IC₅₀ for the inhibition of cell proliferation by the compounds was calculated compared to the DMSO group.

**Table 7. Inhibitory activity of compounds of the present disclosure against Jurkat/293T cell proliferation (IC₅₀, µM)**

| **Compound** | **Jurkat cell** | **293T cell** |
|---|---|---|
| **1** | 0.65 | >10000 |
| **67** | 0.30 | >10000 |
| Reference Compound | 1.94 | >10000 |

### Biological Example 3. Determination of Intracellular Acetyl Lysine/H3K 27 Acetyl Lysine Level with Compound of the Present Disclosure

HeLa cells were seeded into a 96-well plate at 20000 cells/well. After overnight adherence culture, the serially diluted compound was added. After 24 h of treatment, intracellular levels of acetyl lysine and H3K 27 acetyl lysine were quantified by ELISA.

**Table 8. Effect of compound of the present disclosure on levels of acetyl lysine and H3K 27 acetyl lysine**

| **Compound** | **Acetyl lysine** | | **H3K 27 Acetyl lysine** | |
|---|---|---|---|---|
| | Minimum effective concentration | Maximum (%) | Minimum effective concentration | Maximum (%) |
| **1** | 16nM | 500% | 80nM | 200% |
| **67** | 16nM | 570% | 16nM | 300% |
| **Reference Compound** | 80nM | 350% | 80nM | 190% |

### Biological Example 4. Pharmacokinetic Experiment of Compounds of the Present Disclosure in Mice

CD-1 female mice aged 7 to 10 weeks were intravenously administered and orally administered at doses of 1 mg/kg and 10 mg/kg, respectively. The mice were fasted for at least 12 h before the administration and given food 4 h after the administration, and they were given *ad libitum* access to water during the experiment.

On the day of the experiment, animals in the intravenous group were given the corresponding compound by single injection via the tail vein at an administration volume of 10 mL/kg; and animals in the oral group were given the corresponding compound by single intragastric injection at an administration volume of 10 mL/kg. The animals were weighed before administration, and the administration volume was calculated according to the body weight. The sample collection time points were 0.083 h, 0.167 h, 0.5 h, 1 h, 2 h, 4 h, 8 h, and 24 h.

About 200 µL of whole blood was collected through the orbital venous plexus at each time point and used to prepare plasma for concentration determination by high-performance liquid chromatography-tandem mass spectrometry (LC-MS/MS). The plasma concentrations were processed using a non-compartmental model of Winnolin pharmacokinetic software, and the pharmacokinetic parameters were calculated using a linear-log trapezoidal method. The results are shown in Table 13 below.

**Table 9. Results of in vivo pharmacokinetic evaluation of compounds of the present disclosure**

| **Route of Administration** | **Pharmacokinetic parameter** | **Compound 1** |
|---|---|---|
| Injection (1 mg/kg) | Vdss (L/Kg) | 1.68 |
| | T_{1/2} (h) | 3.01 |
| | Cl (mL/min/Kg) | 7.29 |
| | AUC₀₋ₗₐₛₜ (h*ng/mL) | 2278.65 |
| Oral administration (10 mg/kg) | Cₘₐₓ (ng/mL) | 1374.3 |
| | Tₘₐₓ (h) | 2.0 |
| | T_{1/2} (h) | 2.73 |
| | AUC₀₋ₗₐₛₜ (h*ng/mL) | 15359.0 |
| | F% | 67.36% |

### Biological Example 5. Assay for Stability of Compounds of the Present Disclosure in Liver Microsomes

After the 1 µM compound was individually incubated at 37 °C for various durations with 500 mg/mL liver microsomes from humans, monkeys, beagles, rats, or mice in the presence of an NADPH regeneration system, LC-MS-MS was used to analyze the remaining amount of the compound, and T_{1/2} was calculated.

**Table 10. Evaluation results of stability of compounds of the present disclosure in human and mouse liver microsomes**

| **Compound** | **Species** | **T_{1/2} (min)** | **Clint (mL/min/kg)** | **Clₗᵢᵥₑᵣ (mL/min/kg)** |
|---|---|---|---|---|
| **1** | Human | 100.06 | 17.38 | 9.45 |
| | Mouse | 983.61 | *5.55* | 5.23 |
| **3** | Human | 939.33 | 1.85 | 1.70 |
| | Mouse | 306.36 | 17.82 | 14.87 |
| **33** | Human | 210.42 | 8.26 | 5.91 |
| | Mouse | 12039.05 | 0.45 | 0.45 |
| **57** | Human | 231.48 | 7.51 | 5.51 |
| | Mouse | 165.28 | 33.03 | 24.16 |
| **Reference Compound** | Human | 64.92 | 26.78 | 11.68 |
| | Mouse | 532.44 | 10.25 | 9.20 |

As can be seen from the results in the table above, the stability of the compounds of the present disclosure in human liver microsomes was superior to that of the control compound.

### Biological Example 6. In Vivo Efficacy Experiment of Compounds of the Present Disclosure in MC-38 Model

Female C57BL6N mice (aged 6 weeks, 18-22 g) were provided by Vital River Laboratory Animal Technology Co., Ltd. (China) and used one week after quarantine and acclimation. All animals were kept in a room at 23±2 °C with a relative humidity of 50±5%, artificial lighting was provided from 08:00 to 20:00 every day, and the air was exchanged 13-18 times per hour.

Mouse colon cancer MC38 cells were cultured conventionally in 1640 medium containing 10% fetal bovine serum in an incubator at 37 °C with 5% CO₂. After being subcultured, the cells were collected when they reached the desired amount. The C57BL6N mice were subcutaneously injected with 2 × 10⁶ MC38 cells on the right side to form tumors. After the tumors grew to about 100 mm³, the animals were randomly grouped for administration. Tumor volumes were measured with a caliper on day 3, day 7, day 10, day 14, day 17, and day 21 after the administration. The ability of the compounds to inhibit tumor growth was evaluated according to the tumor growth inhibition rate (TGI) = 1 - (tumor volume on day 28 in treatment group - tumor volume on day 1 in treatment group)/(tumor volume on day 28 in control group - tumor volume on day 1 in control group). The toxicity of the compounds was evaluated according to the body weight and state of the mice.

The groups were as follows:
1) solvent control group; 2) PD-1+Reg group; 3) Compound 1 group; 4) Reference Compound group; 5) compound 1 (10mg/kg) and PD-1+Reg Combination group; 6) compound 1 (30mg/kg) and PD-1+Reg Combination group; 7) compound 1 (90mg/kg) and PD-1+Reg Combination group; 8) Reference Compound (10mg/kg) and PD-1+Reg Combination group; 9) Reference Compound (30mg/kg) and PD-1+Reg Combination group; 10) Reference Compound (90mg/kg) and PD-1+Reg Combination group, with 6 mice per group. The results are shown in Table 11 below.

**Table 11. In vivo efficacy of some of the compounds of the present disclosure in MC-38 model**

| **Gr oup** | **Compound** | **Dose (mg/kg)** | **Administra tion period *** | **Route of Administra tion** | **TGI(%)** | **Change in body weight(%)** |
|---|---|---|---|---|---|---|
| **1** | Solvent control group | -- | QD | IV+PO+PO | -- | 15.03+3.23 |
| **2** | PD-1+Reg | 3+5 | QW+QD | IV+PO | 81.55 | 11.23±2.8 |
| **3** | Compound 1 | 90 | QD | PO | 100.54 (5CR) | 11.08+1.08 |
| **4** | Reference Compund | 90 | QD | PO | 73.90 (1PR) | 17.04+1.54 |
| **5** | Compound 1 + PD-1+Reg | 10 +3+5 | QD +QW+QD | PO +IV+PO | 102.04 (5CR) | 6.59+1.74 |
| **6** | Compound 1 + PD-1+Reg | 30 +3+5 | QD +QW+QD | PO +IV+PO | 96.97 (4CR+1SD) | 8.57+1.89 |
| **7** | Compound 1 + PD-1+Reg | 90 +3+5 | QD +QW+QD | PO +IV+PO | 103.35 (5CR+1died) | 11.43+1.89 |
| **8** | Reference Compund +PD-1+Reg | 10 +3+5 | QD +QW+QD | PO +IV+PO | 94.48 (3CR) | 6.42+2.14 |
| **9** | Reference Compund +PD-1+Reg | 30 +3+5 | QD +QW+QD | PO +IV +PO | 99.77 (2CR+1PR+2SD) | 8.17±1.39 |
| **10** | Reference Compund +PD-1+Reg | 90 +3+5 | QD +QW+QD | PO +IV+PO | 102.26 (4CR+1PR+1SD) | 7.99±1.47 |

Reg represents Regorafenib PD-1 represents an anti-PD-1 antibody; IV represents administration via intravenous injection; PO represents oral administration; QD represents once-daily administration; QW represents once-weekly administration; CR represents complete remission (complete disappearance of the tumor after the dosing cycle is completed); PR represents partial response (tumor volume reduction exceeding 30% relative to the initial volume upon completion of the administration cycle); SD represents stable disease (tumor volume reduction or growth not exceeding 30% relative to the initial volume upon completion of the administration cycle); Died represents mouse died before the dosing cycle is completed; Administration period* is once daily (QD) for 10 consecutive days, followed by a 16-day off-treatment period; the once-weekly (QW) regimen is administered only on Day 1 and Day 8.

As can be seen from the results of the *in vivo* experiment described above, the compounds of the present disclosure, whether as monotherapy or in combination with PD-1 and Reg, exhibit marked inhibitory effect on the MC-38 *in vivo* tumor model. Specifically, the Compound 1 monotherapy group achieved complete tumor regression in 83.3% (5/6) of mice, while the triple combination of Compound 1 + PD-1 + Reg produced complete regression in 83.3% (5/6) of mice at a lower dose and 100% (5/5) at a higher dose. Compared with the reference compound, at equivalent doses both the monotherapy and the triple regimen show a clear advantage in suppressing tumor growth.

### Biological Example 7. In Vivo Efficacy Experiment of Compounds of the Present Disclosure in CT-26 Model

Female BALB/c mice (aged 6 weeks, 18-22 g) were provided by Vital River Laboratory Animal Technology Co., Ltd. (China) and used one week after quarantine and acclimation. All animals were kept in a room at 23±2 °C with a relative humidity of 50±5%, artificial lighting was provided from 08:00 to 20:00 every day, and the air was exchanged 13-18 times per hour.

Mouse colon cancer CT-26 cells were cultured conventionally in 1640 medium containing 10% fetal bovine serum in an incubator at 37 °C with 5% CO₂. After being subcultured, the cells were collected when they reached the desired amount. The BALB/c mice were subcutaneously injected with 2 × 10⁵ CT-26 cells on the right side to form tumors. After the tumors grew to about 100 mm³, the animals were randomly grouped for administration. Tumor volumes were measured with a caliper on day 3, day 7, day 10, day 14, day 17, and day 21 after the administration. The ability of the compounds to inhibit tumor growth was evaluated according to the tumor growth inhibition rate (TGI) = 1 - (tumor volume on day 28 in treatment group - tumor volume on day 1 in treatment group)/(tumor volume on day 28 in control group - tumor volume on day 1 in control group). The toxicity of the compounds was evaluated according to the body weight and state of the mice.

The groups were as follows:
1) solvent control group; 2) PD-1+Reg group; 3) Compound 1-A (30mg/kg) group; 4) Compound 1-B (90mg/kg) group; 5) Reference Compound-A (30mg/kg) group; 6) Reference Compound-B (90mg/kg) group; 7) compound 1 (10mg/kg) and PD-1+Reg Combination group; 8) compound 1 (30mg/kg) and PD-1+Reg Combination group; 9) compound 1 (90mg/kg) and PD-1+Reg Combination group; 10) Reference Compund (10mg/kg) and PD-1+Reg Combination group; 11) Reference Compund (30mg/kg) and PD-1+Reg Combination group; 12) Reference Compund (90mg/kg) and PD-1+Reg Combination group, with 6 mice per group. The results are shown in Table 12 below.

**Table 12. In vivo efficacy of some of the compounds of the present disclosure in CT-26 model**

| **Gro up** | **Compound** | **Dose (mg/kg)** | **Administra tion period *** | **Route of Administra tion** | **TGI(%)** | **Change in body weight(%)** |
|---|---|---|---|---|---|---|
| **1** | Solvent control group | -- | QD | IV+PO+PO | -- | 9.22+1.56 |
| **2** | PD-1+Reg | 3+5 | QW+QD | IV+PO | 64.95 (2PR) | 3.37±2.8 |
| **3** | Compound 1-A | 30 | QD | PO | 38.57 (1PR) | 1.13+3.79 |
| **4** | Compound 1-B | 90 | QD | PO | 88.35 (1PR+1SD) | -10.53+7.1 |
| **5** | Reference Compund -A | 30 | QD | PO | 56.10 (1PR) | 4.73±1.54 |
| **6** | Reference Compund -B | 90 | QD | PO | 95.93 (1PR+2SD) | -3.53±2.89 |
| **7** | Compound 1 +PD-1+Reg | 10 +3+5 | QD +QW+QD | PO +IV+PO | 81.48 (3PR) | -4.08+1.13 |
| **8** | Compound 1 +PD-1+Reg | 30 +3+5 | QD +QW+QD | PO +IV+PO | 102.08 (4PR+1SD) | -0.92+0.56 |
| **9** | Compound 1 +PD-1+Reg | 90 +3+5 | QD +QW+QD | PO +IV+PO | 101.81 (5PR) | -12.52+3.49 |
| **10** | Reference Compund +PD-1+Reg | 10 +3+5 | QD +QW+QD | PO +IV+PO | 86.36 (3PR) | 0.11±1.35 |
| **11** | Reference Compund +PD-1+Reg | 30 +3+5 | QD +QW+QD | PO +IV+PO | 88.35 (4PR) | -0.97+1.88 |
| **12** | Reference Compund +PD-1+Reg | 90 +3+5 | QD +QW+QD | PO +IV+PO | 92.23 (2PR+2SD) | -3.57+2.98 |

Reg represents Regorafenib PD-1 represents an anti-PD-1 antibody; IV represents administration via intravenous injection; PO represents oral administration; QD represents once-daily administration; QW represents once-weekly administration; CR represents complete remission (complete disappearance of the tumor after the dosing cycle is completed); PR represents partial response (tumor volume reduction exceeding 30% relative to the initial volume upon completion of the administration cycle); SD represents stable disease (tumor volume reduction or growth not exceeding 30% relative to the initial volume upon completion of the administration cycle); administration period is once daily (QD) for 14 consecutive days; the once-weekly (QW) regimen is administered only on Day 1 and Day 8.

As can be seen from the results of the *in vivo* experiment described above, the compounds of the present disclosure, when administered in combination with PD-1 and Reg, exhibited a relatively good inhibitory effect on the CT-26 *in vivo* tumor model. Among them, Group 7 induced marked tumor shrinkage in 50% (3/6) of mice, Group 8 in 66.7% (4/6), and Group 9 in 83.3% (5/6). At equivalent doses, the triple combination of the compounds of the present disclosure outperformed the reference compound.

### Biological Example 8. PBMC-Mediated Tumor Cell Killing by Compounds of the Present Disclosure

OVCAR 3 cells were seeded into a 96-well plate at 4000 cells/well. After overnight adherence culture, the serially diluted compounds were added, and the plate was incubated for another 72 h. After OVCAR 3 cells were stained with Calcein AM, PBMCs were added. The cells were cultured for another 1-4 h and washed with PBS, and tumor cells were counted. The EC₅₀ of the compounds in promoting cell death was calculated relative to DMSO.

### Biological Example 9. Assay for PD-L1 Expression in Cells by Compounds of the Present Disclosure

MD-MBA 231 cells were seeded into a 96-well plate at 3000 cells/well. After overnight adherence culture, serially diluted compounds were added. After 72 h of treatment, the expression of PD-L1 on the cell surface was detected by ELISA.

Although specific embodiments of the present disclosure have been described above, it will be appreciated by those skilled in the art that these embodiments are merely illustrative and that many changes or modifications can be made to these embodiments without departing from the principles and spirit of the present disclosure.

## Claims

1. A compound represented by general formula (1) or an isomer thereof, a crystalline form thereof, a pharmaceutically acceptable salt thereof, a hydrate thereof, or a solvate thereof: 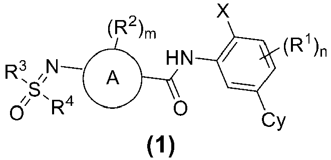 wherein in general formula (1):
X is -NH₂ or -OH;
Cy is (C6-C14) aryl or (5-14 membered) heteroaryl, wherein the (C6-C14) aryl or (5-14 membered) heteroaryl may be each independently and optionally substituted with 1, 2, 3, or 4 R⁵
ring A is (C6-C14) aryl, (5-14 membered) heteroaryl, (C3-C14) cycloalkyl, or (3-14 membered) heterocycloalkyl;
R¹ is H, D, or halogen;
R² is H, D, halogen, (C1-C4) alkyl, (C1-C4) alkoxy, (C1-C4) haloalkyl, (C2-C4) alkenyl, (C2-C4) alkynyl, (C3-C6) cycloalkyl, or (3-6 membered) heterocycloalkyl;
R³ is (C1-C6) alkyl, (C1-C6) alkoxy, (C1-C6) haloalkyl, (C2-C4) alkenyl, (C2-C4) alkynyl, (C3-C6) cycloalkyl, or (3-6 membered) heterocycloalkyl, wherein the (C1-C6) alkyl, (C1-C6) alkoxy, (C1-C6) haloalkyl, (C2-C4) alkenyl, (C2-C4) alkynyl, (C3-C6) cycloalkyl, or (3-6 membered) heterocycloalkyl may be each independently and optionally substituted with 1, 2, 3, or 4 R^{a};
R⁴ is (C1-C6) alkyl, (C1-C6) alkoxy, (C1-C6) haloalkyl, (C2-C4) alkenyl, (C2-C4) alkynyl, (C3-C6) cycloalkyl, or (3-6 membered) heterocycloalkyl, wherein the (C1-C6) alkyl, (C1-C6) alkoxy, (C1-C6) haloalkyl, (C2-C4) alkenyl, (C2-C4) alkynyl, (C3-C6) cycloalkyl, or (3-6 membered) heterocycloalkyl may be each independently and optionally substituted with 1, 2, 3, or 4 R^{b};
or R³ and R⁴, together with the S atom to which they are attached, form (3-16 membered) heterocycloalkyl, wherein the (3-16 membered) heterocycloalkyl may be each independently and optionally substituted with 1, 2, 3, or 4 R⁶;
R⁵ is H, D, halogen, hydroxy, amino, cyano, nitro, -OR^{a}, -NR^{a}R^{b}, -C(O)R^{a}, -COzR^{a}, -(CH₂)mR^{a}, -(CH₂)mCO₂R^{a}, -(CH₂)ₘ-CONR^{a}R^{b}, -S(O)ₚR^{a}, -S(O)ₚNR^{a}R^{b}, -CONR^{a}R^{b}, -C(=NR^{a})-NR^{a}R^{b}, -NR^{a}COR^{b}, -CR^{a}CONR^{a}R^{b}, - NR^{a}CO₂R^{a}, -NR^{a}S(O)ₚNR^{a}R^{b}, -NR^{a}S(O)ₚR^{a}, -P(O)ₚR^{a}R^{b}, (C1-C8) alkyl, (C1-C8) alkoxy, (C1-C8) haloalkyl, (C2-C8) alkenyl, (C2-C8) alkynyl, (C3-C14) cycloalkyl, (3-14 membered) heterocycloalkyl, (C6-C14) aryl, (5-14 membered) heteroaryl, -(C1-C8) alkylene-(C3-C14) cycloalkyl, -(C1-C8) alkylene-(3-14 membered) heterocycloalkyl, -(C1-C8) alkylene-(C6-C14) aryl, or -(C1-C8) alkylene-(5-14 membered) heteroaryl, wherein the (C1-C8) alkyl, (C1-C8) alkoxy, (C1-C8) haloalkyl, (C2-C8) alkenyl, (C2-C8) alkynyl, (C3-C14) cycloalkyl, (3-14 membered) heterocycloalkyl, (C6-C14) aryl, (5-14 membered) heteroaryl, -(C1-C8) alkylene-(C3-C14) cycloalkyl, -(C1-C8) alkylene-(3-14 membered) heterocycloalkyl, -(C1-C8) alkylene-(C6-C14) aryl, or -(C1-C8) alkylene-(5-14 membered) heteroaryl may be each independently and optionally substituted with 1, 2, 3, or 4 R^{c};
R⁶ is H, D, halogen, hydroxy, amino, cyano, nitro, -OR^{a}, -NR^{a}R^{b}, -C(O)R^{a}, -COzR^{a}, -(CH₂)mR^{a}, -(CH₂)mCO₂R^{a}, -(CH₂)ₘ-CONR^{a}R^{b}, -S(O)ₚR^{a}, -S(O)ₚNR^{a}R^{b}, -CONR^{a}R^{b}, -C(=NR^{a})-NR^{a}R^{b}, -NR^{a}COR^{b}, -CR^{a}CONR^{a}R^{b}, - NR^{a}CO₂R^{a}, -NR^{a}S(O)ₚNR^{a}R^{b}, -NR^{a}S(O)ₚR^{a}, -P(O)ₚR^{a}R^{b}, (C1-C8) alkyl, (C1-C8) alkoxy, (C1-C8) haloalkyl, (C2-C8) alkenyl, (C2-C8) alkynyl, (C3-C14) cycloalkyl, (3-14 membered) heterocycloalkyl, (C6-C14) aryl, (5-14 membered) heteroaryl, -(C1-C8) alkylene-(C3-C14) cycloalkyl, -(C1-C8) alkylene-(3-14 membered) heterocycloalkyl, -(C1-C8) alkylene-(C6-C14) aryl, or -(C1-C8) alkylene-(5-14 membered) heteroaryl, wherein the (C1-C8) alkyl, (C1-C8) alkoxy, (C1-C8) haloalkyl, (C2-C8) alkenyl, (C2-C8) alkynyl, (C3-C14) cycloalkyl, (3-14 membered) heterocycloalkyl, (C6-C14) aryl, (5-14 membered) heteroaryl, -(C1-C8) alkylene-(C3-C14) cycloalkyl, -(C1-C8) alkylene-(3-14 membered) heterocycloalkyl, -(C1-C8) alkylene-(C6-C14) aryl, or -(C1-C8) alkylene-(5-14 membered) heteroaryl may be each independently and optionally substituted with 1, 2, 3, or 4 R^{c}; or when 2 R⁶ are attached to the same atom, the 2 R⁶, together with the atom to which they are attached, may form one oxo group, (C3-C6) cycloalkyl group, or (3-6 membered) heterocycloalkyl group;
R^{a} and R^{b} are each independently -H, -D, halogen, hydroxy, amino, cyano, nitro, (C1-C8) alkyl, (C1-C8) alkoxy, (C1-C8) haloalkyl, (C2-C8) alkenyl, (C2-C8) alkynyl, (C3-C14) cycloalkyl, (3-14 membered) heterocycloalkyl, (C6-C14) aryl, (5-14 membered) heteroaryl, -(C1-C8) alkylene-(C3-C14) cycloalkyl, -(C1-C8) alkylene-(3-14 membered) heterocycloalkyl, -(C1-C8) alkylene-(C6-C14) aryl, or -(C1-C8) alkylene-(5-14 membered) heteroaryl, wherein the (C1-C8) alkyl, (C1-C8) alkoxy, (C1-C8) haloalkyl, (C2-C8) alkenyl, (C2-C8) alkynyl, (C3-C14) cycloalkyl, (3-14 membered) heterocycloalkyl, (C6-C14) aryl, (5-14 membered) heteroaryl, -(C1-C8) alkylene-(C3-C14) cycloalkyl, -(C1-C8) alkylene-(3-14 membered) heterocycloalkyl, - (C1-C8) alkylene-(C6-C14) aryl, or -(C1-C8) alkylene-(5-14 membered) heteroaryl may be each independently and optionally substituted with 1, 2, 3, or 4 R^{c}; or when 2 R^{a} are attached to the same atom, the 2 R^{a} may form one oxo group; or when 2 R^{b} are attached to the same atom, the 2 R^{b} may form one oxo group; or R^{a} and R^{b} attached to the same atom, together with the atom to which they are attached, form one (5-7 membered) heterocycloalkyl group or (C3-C9) cycloalkyl group, wherein the (5-7 membered) heterocycloalkyl group or (C3-C9) cycloalkyl group is each independently and optionally substituted with 1, 2, 3, or 4 R^{c};
R^{c} is -H, -D, halogen, hydroxy, amino, cyano, nitro, (C1-C8) alkyl, (C1-C8) alkoxy, (C1-C8) haloalkyl, (C2-C8) alkenyl, (C2-C8) alkynyl, (C3-C14) cycloalkyl, (3-14 membered) heterocycloalkyl, (C6-C14) aryl, (5-14 membered) heteroaryl, -(C1-C8) alkylene-(C1-C8) alkoxy, -(C1-C8) alkylene-(C3-C14) cycloalkyl, -(C1-C8) alkylene-(3-14 membered) heterocycloalkyl, -(C1-C8) alkylene-(C6-C14) aryl, or -(C1-C8) alkylene-(5-14 membered) heteroaryl; or when 2 R^{c} are attached to the same atom, the 2 R^{c} may form one oxo group, (C3-C6) cycloalkyl group, or (3-6 membered) heterocycloalkyl group;
m, n, and p are integers of 0, 1, or 2.

2. The compound or the isomer thereof, the crystalline form thereof, the pharmaceutically acceptable salt thereof, the hydrate thereof, or the solvate thereof according to claim 1, wherein in general formula (1), X is - NH₂ or -OH; preferably, X is -NH₂.

3. The compound or the isomer thereof, the crystalline form thereof, the pharmaceutically acceptable salt thereof, the hydrate thereof, or the solvate thereof according to claim 1 or 2, wherein in general formula (1), ring A is (C6-C10) aryl, (5-10 membered) heteroaryl, (C3-C12) cycloalkyl, or (3-12 membered) heterocycloalkyl; preferably, ring A is phenyl, (5-7 membered) heteroaryl, or (C5-C7) cycloalkyl; more preferably, ring A is phenyl, pyridinyl, or cyclohexyl.

4. The compound or the isomer thereof, the crystalline form thereof, the pharmaceutically acceptable salt thereof, the hydrate thereof, or the solvate thereof according to any one of claims 1-3, wherein in general formula (1), Cy is (C6-C10) aryl or (5-12 membered) heteroaryl, wherein the (C6-C10) aryl or (5-12 membered) heteroaryl may be each independently and optionally substituted with 1, 2, 3, or 4 R⁵; preferably, Cy is phenyl or (5-7 membered) heteroaryl.

5. The compound or the isomer thereof, the crystalline form thereof, the pharmaceutically acceptable salt thereof, the hydrate thereof, or the solvate thereof according to claim 4, wherein in general formula (1), Cy is phenyl or (5-6 membered) heteroaryl, preferably

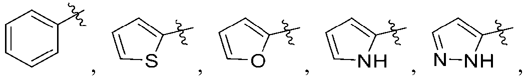

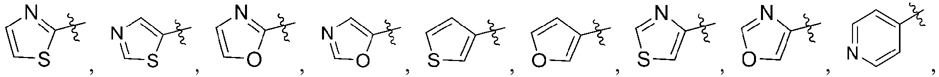

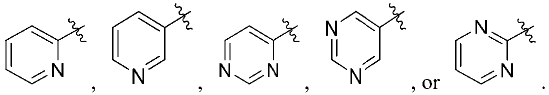

6. The compound or the isomer thereof, the crystalline form thereof, the pharmaceutically acceptable salt thereof, the hydrate thereof, or the solvate thereof according to claim 4 or 5, wherein in general formula (1), R⁵ is H, D, halogen, hydroxy, amino, cyano, nitro, -OR^{a}, -NR^{a}R^{b}, -C(O)R^{a}, -COzR^{a}, -(CH₂)mR^{a}, -(CH₂)mCO₂R^{a}, -(CH₂)ₘCONR^{a}R^{b}, -S(O)ₚR^{a}, -S(O)ₚNR^{a}R^{b}, -CONR^{a}R^{b}, -C(=NR^{a})-NR^{a}R^{b}, -NR^{a}COR^{b}, -CR^{a}CONR^{a}R^{b}, - NR^{a}CO₂R^{a}, -NR^{a}S(O)ₚNR^{a}R^{b}, -NR^{a}S(O)ₚR^{a}, -P(O)pR^{a}R^{b}, (C1-C6) alkyl, (C1-C6) alkoxy, (C1-C6) haloalkyl, (C2-C6) alkenyl, (C2-C6) alkynyl, (C3-C12) cycloalkyl, (3-12 membered) heterocycloalkyl, (C6-C14) aryl, (5-14 membered) heteroaryl, -(C1-C6) alkylene-(C3-C14) cycloalkyl, -(C1-C6) alkylene-(3-14 membered) heterocycloalkyl, -(C1-C6) alkylene-(C6-C14) aryl, or -(C1-C6) alkylene-(5-14 membered) heteroaryl, wherein the (C1-C6) alkyl, (C1-C6) alkoxy, (C1-C6) haloalkyl, (C2-C6) alkenyl, (C2-C6) alkynyl, (C3-C12) cycloalkyl, (3-12 membered) heterocycloalkyl, (C6-C14) aryl, (5-14 membered) heteroaryl, -(C1-C6) alkylene-(C3-C14) cycloalkyl, -(C1-C6) alkylene-(3-14 membered) heterocycloalkyl, -(C1-C6) alkylene-(C6-C14) aryl, or -(C1-C6) alkylene-(5-14 membered) heteroaryl may be each independently and optionally substituted with 1, 2, 3, or 4 R^{c}; preferably, R⁵ is H, D, halogen, hydroxy, amino, cyano, nitro, -OR^{a}, -NR^{a}R^{b}, -CONR^{a}R^{b}, -P(O)pR^{a}R^{b}, -S(O)ₚR^{a}, (C1-C4) alkyl, (C1-C4) alkoxy, (C1-C4) haloalkyl, (C2-C4 alkenyl, (C2-C4) alkynyl, (C3-C7) cycloalkyl, or (3-7 membered) heterocycloalkyl.

7. The compound or the isomer thereof, the crystalline form thereof, the pharmaceutically acceptable salt thereof, the hydrate thereof, or the solvate thereof according to claim 6, wherein in general formula (1), R⁵ is H, D, F, Cl, -CN, (C1-C3) alkyl, (C1-C3) alkoxy, (C1-C3) haloalkyl, or (C3-C6) cycloalkyl; preferably, R⁵ is H, D, F, Cl, -CN, -CH₃, -CF₃, -OCH₃, or

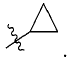

8. The compound or the isomer thereof, the crystalline form thereof, the pharmaceutically acceptable salt thereof, the hydrate thereof, or the solvate thereof according to any one of claims 1-7, wherein in general formula (1), R¹ is H, D, F, Cl, Br, or I; preferably, R¹ is H, D, or F.

9. The compound or the isomer thereof, the crystalline form thereof, the pharmaceutically acceptable salt thereof, the hydrate thereof, or the solvate thereof according to any one of claims 1-8, wherein in general formula (1), R² is H, D, F, Cl, Br, I, (C1-C3) alkyl, (C1-C3) alkoxy, (C1-C3) haloalkyl, (C2-C4) alkenyl, (C2-C4) alkynyl, (C3-C6) cycloalkyl, or (3-6 membered) heterocycloalkyl; preferably, R² is H, D, or F.

10. The compound or the isomer thereof, the crystalline form thereof, the pharmaceutically acceptable salt thereof, the hydrate thereof, or the solvate thereof according to any one of claims 1-9, wherein in general formula (1), R³ is (C1-C4) alkyl, (C1-C4) alkoxy, (C1-C4) haloalkyl, (C2-C4) alkenyl, (C2-C4) alkynyl, (C3-C6) cycloalkyl, or (3-6 membered) heterocycloalkyl, wherein the (C1-C4) alkyl, (C1-C4) alkoxy, (C1-C4) haloalkyl, (C2-C4) alkenyl, (C2-C4) alkynyl, (C3-C6) cycloalkyl, or (3-6 membered) heterocycloalkyl may be each independently and optionally substituted with 1, 2, 3, or 4 R^{a}; preferably, R³ is (C1-C4) alkyl, (C1-C4) alkoxy, or (C1-C4) haloalkyl; more preferably, R³ is methyl, ethyl, or methoxymethyl.

11. The compound or the isomer thereof, the crystalline form thereof, the pharmaceutically acceptable salt thereof, the hydrate thereof, or the solvate thereof according to any one of claims 1-10, wherein in general formula (1), R⁴ is (C1-C4) alkyl, (C1-C4) alkoxy, (C1-C4) haloalkyl, (C2-C4) alkenyl, (C2-C4) alkynyl, (C3-C6) cycloalkyl, or (3-6 membered) heterocycloalkyl, wherein the (C1-C4) alkyl, (C1-C4) alkoxy, (C1-C4) haloalkyl, (C2-C4) alkenyl, (C2-C4) alkynyl, (C3-C6) cycloalkyl, or (3-6 membered) heterocycloalkyl may be each independently and optionally substituted with 1, 2, 3, or 4 R^{b}; preferably, R⁴ is (C1-C4) alkyl, (C1-C4) alkoxy, or (C1-C4) haloalkyl; more preferably, R⁴ is methyl, ethyl, or methoxymethyl.

12. The compound or the isomer thereof, the crystalline form thereof, the pharmaceutically acceptable salt thereof, the hydrate thereof, or the solvate thereof according to any one of claims 1-9, wherein in general formula (1), R³ and R⁴, together with the S atom to which they are attached, form (3-14 membered) heterocycloalkyl, wherein the (3-14 membered) heterocycloalkyl may be each independently and optionally substituted with 1, 2, 3, or 4 R⁶.

13. The compound or the isomer thereof, the crystalline form thereof, the pharmaceutically acceptable salt thereof, the hydrate thereof, or the solvate thereof according to claim 12, wherein in general formula (1), R³ and R⁴, together with the S atom to which they are attached, form one (4-6 membered) monocyclic heterocycloalkyl group containing 1 or 2 atoms optionally selected from N, S, and O, or (7-9 membered) bicyclic spiro heterocycloalkyl group containing 1 or 2 atoms optionally selected from N, S, and O, wherein the (4-6 membered) monocyclic heterocycloalkyl group or (7-9 membered) bicyclic spiro heterocycloalkyl group may be each independently and optionally substituted with 1, 2, 3, or 4 R⁶.

14. The compound or the isomer thereof, the crystalline form thereof, the pharmaceutically acceptable salt thereof, the hydrate thereof, or the solvate thereof according to claim 12 or 13, wherein in general formula (1), R⁶ is H, D, halogen, hydroxy, amino, cyano, nitro, -OR^{a}, -NR^{a}R^{b}, -C(O)R^{a}, -COzR^{a}, -(CH₂)mR^{a}, -(CH₂)mCO₂R^{a}, -(CH₂)ₘCONR^{a}R^{b}, -S(O)ₚR^{a}, -S(O)ₚNR^{a}R^{b}, -CONR^{a}R^{b}, -C(=NR^{a})-NR^{a}R^{b}, -NR^{a}COR^{b}, -CR^{a}CONR^{a}R^{b}, - NR^{a}CO₂R^{a}, -NR^{a}S(O)ₚNR^{a}R^{b}, -NR^{a}S(O)ₚR^{a}, -P(O)pR^{a}R^{b}, (C1-C6) alkyl, (C1-C6) alkoxy, (C1-C6) haloalkyl, (C2-C6) alkenyl, (C2-C6) alkynyl, (C3-C14) cycloalkyl, (3-14 membered) heterocycloalkyl, (C6-C14) aryl, (5-14 membered) heteroaryl, -(C1-C6) alkylene-(C3-C14) cycloalkyl, -(C1-C6) alkylene-(3-14 membered) heterocycloalkyl, -(C1-C6) alkylene-(C6-C14) aryl, or -(C1-C6) alkylene-(5-14 membered) heteroaryl, wherein the (C1-C6) alkyl, (C1-C6) alkoxy, (C1-C6) haloalkyl, (C2-C6) alkenyl, (C2-C6) alkynyl, (C3-C14) cycloalkyl, (3-14 membered) heterocycloalkyl, (C6-C14) aryl, (5-14 membered) heteroaryl, -(C1-C6) alkylene-(C3-C14) cycloalkyl, -(C1-C6) alkylene-(3-14 membered) heterocycloalkyl, -(C1-C6) alkylene-(C6-C14) aryl, or -(C1-C6) alkylene-(5-14 membered) heteroaryl may be each independently and optionally substituted with 1, 2, 3, or 4 R^{c}; preferably, R⁶ is H, D, halogen, hydroxy, amino, cyano, -OR^{a}, -NR^{a}R^{b}, (C1-C4) alkyl, (C1-C4) alkoxy, (C1-C4) haloalkyl, -(C1-C4) alkylene-(C3-C7) cycloalkyl, -(C1-C4) alkylene-(3-7 membered) heterocycloalkyl, or -(C1-C4) alkylene-(5-7 membered) heteroaryl, wherein the (C1-C4) alkyl, (C1-C4) alkoxy, (C1-C4) haloalkyl, -(C1-C4) alkylene-(C3-C7) cycloalkyl, -(C1-C4) alkylene-(3-7 membered) heterocycloalkyl, or -(C1-C4) alkylene-(5-7 membered) heteroaryl may be each independently and optionally substituted with 1, 2, 3, or 4 R^{c}; or when 2 R⁶ are attached to the same atom, the 2 R⁶, together with the atom to which they are attached, may form one oxo group.

15. The compound or the isomer thereof, the crystalline form thereof, the pharmaceutically acceptable salt thereof, the hydrate thereof, or the solvate thereof according to claim 14, wherein in general formula (1), R⁶ is H, D, F, Cl, hydroxy, amino, cyano, methyl, ethyl, -NH(CH₃), -N(CH₃)₂, -CD₃, -C(O)CH₃, or

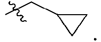

16. The compound or the isomer thereof, the crystalline form thereof, the pharmaceutically acceptable salt thereof, the hydrate thereof, or the solvate thereof according to any one of claims 1-15, wherein in general formula (1), R^{a} and R^{b} are each independently -H, -D, halogen, hydroxy, amino, cyano, nitro, (C1-C6) alkyl, (C1-C6) alkoxy, (C1-C6)haloalkyl, (C2-C6) alkenyl, (C2-C6) alkynyl, (C3-C14) cycloalkyl, (3-14 membered) heterocycloalkyl, (C6-C14) aryl, (5-14 membered) heteroaryl, -(C1-C6) alkylene-(C3-C14) cycloalkyl, -(C1-C6) alkylene-(3-14 membered) heterocycloalkyl, -(C1-C6) alkylene-(C6-C14) aryl, or -(C1-C6) alkylene-(5-14 membered) heteroaryl, wherein the (C1-C6) alkyl, (C1-C6) alkoxy, (C1-C6) haloalkyl, (C2-C6) alkenyl, (C2-C6) alkynyl, (C3-C14) cycloalkyl, (3-14 membered) heterocycloalkyl, (C6-C14) aryl, (5-14 membered) heteroaryl, -(C1-C6) alkylene-(C3-C14) cycloalkyl, -(C1-C6) alkylene-(3-14 membered) heterocycloalkyl, - (C1-C6) alkylene-(C6-C14) aryl, or -(C1-C6) alkylene-(5-14 membered) heteroaryl may be each independently and optionally substituted with 1, 2, 3, or 4 R^{c}; or when 2 R^{a} are attached to the same atom, the 2 R^{a} may form one oxo group; or when 2 R^{b} are attached to the same atom, the 2 R^{b} may form one oxo group; preferably, R^{a} and R^{b} are each independently H, (C1-C4) alkyl, (C1-C4) alkoxy, (C1-C4) haloalkyl, (C3-C7) cycloalkyl, (3-7 membered) heterocycloalkyl, (C6-C10) aryl, or (5-7 membered) heteroaryl, wherein the (C1-C4) alkyl, (C1-C4) alkoxy, (C1-C4) haloalkyl, (C3-C7) cycloalkyl, (3-7 membered) heterocycloalkyl, (C6-C10) aryl, or (5-7 membered) heteroaryl may be each independently and optionally substituted with 1, 2, 3, or 4 R^{c}.

17. The compound or the isomer thereof, the crystalline form thereof, the pharmaceutically acceptable salt thereof, the hydrate thereof, or the solvate thereof according to any one of claims 1-15, wherein in general formula (1), R^{a} and R^{b} attached to the same atom, together with the atom to which they are attached, form one (5-7 membered) heterocycloalkyl group or (C3-C9) cycloalkyl group, wherein the (5-7 membered) heterocycloalkyl group or (C3-C9) cycloalkyl group is each independently and optionally substituted with 1, 2, 3, or 4 R^{c}.

18. The compound or the isomer thereof, the crystalline form thereof, the pharmaceutically acceptable salt thereof, the hydrate thereof, or the solvate thereof according to any one of claims 1-17, wherein in general formula (1), R^{c} is -H, -D, halogen, hydroxy, amino, cyano, nitro, (C1-C6) alkyl, (C1-C6) alkoxy, (C1-C6) haloalkyl, (C2-C6) alkenyl, (C2-C6) alkynyl, (C3-C14) cycloalkyl, (3-14 membered) heterocycloalkyl, (C6-C14) aryl, (5-14 membered) heteroaryl, -(C1-C6) alkylene-(C1-C8) alkoxy, -(C1-C6) alkylene-(C3-C14) cycloalkyl, -(C1-C6) alkylene-(3-14 membered) heterocycloalkyl, -(C1-C6) alkylene-(C6-C14) aryl, or -(C1-C6) alkylene-(5-14 membered) heteroaryl; or when 2 R^{c} are attached to the same atom, the 2 R^{c} may form one oxo group; preferably, R^{c} is H, D, halogen, hydroxy, amino, cyano, (C1-C4) alkyl, (C1-C4) alkoxy, (C1-C4) haloalkyl, (C3-C7) cycloalkyl, or (3-7 membered) heterocycloalkyl.

19. The compound or the isomer thereof, the crystalline form thereof, the pharmaceutically acceptable salt thereof, the hydrate thereof, or the solvate thereof according to claims 1-18, wherein the compound has one of the following structures:

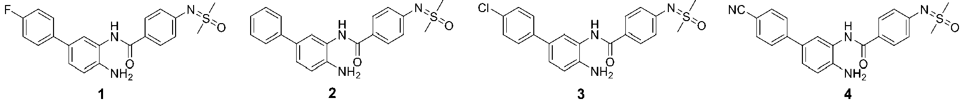

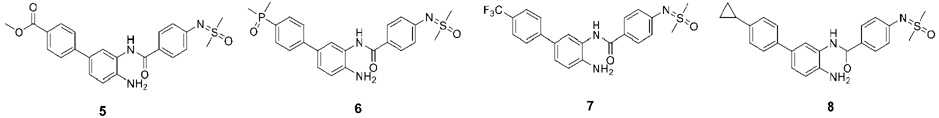

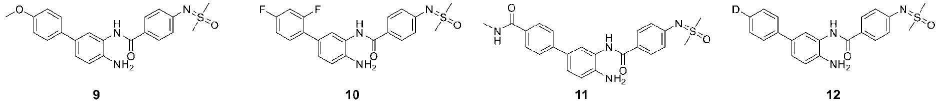

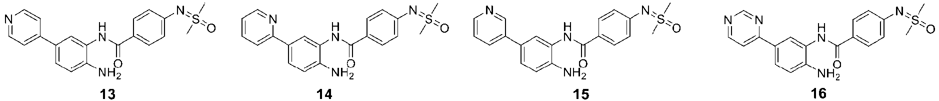

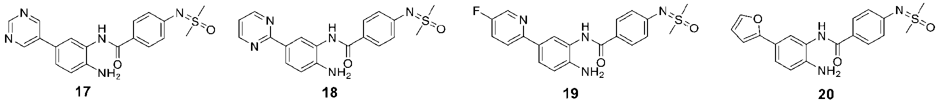

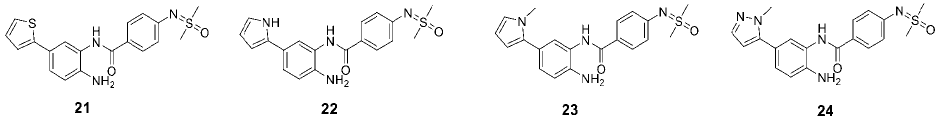

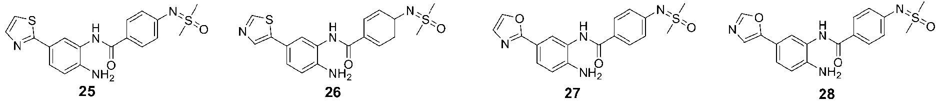

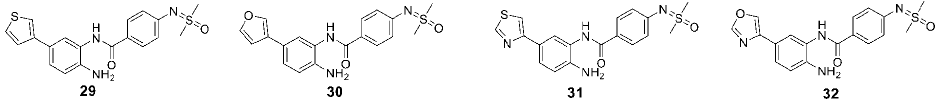

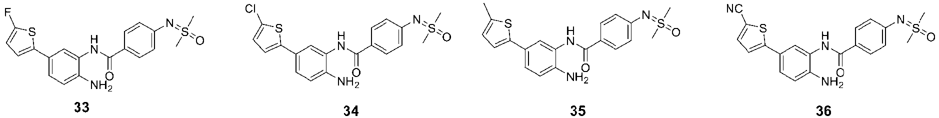

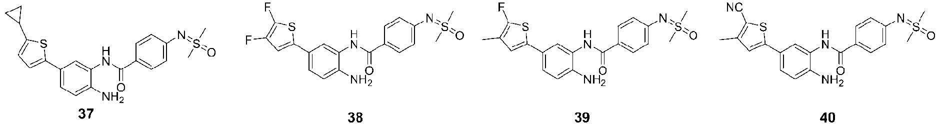

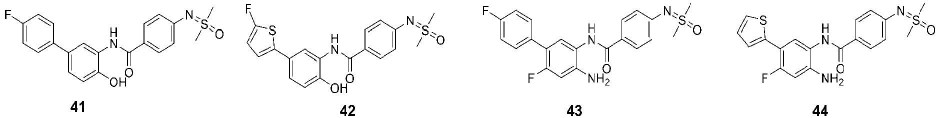

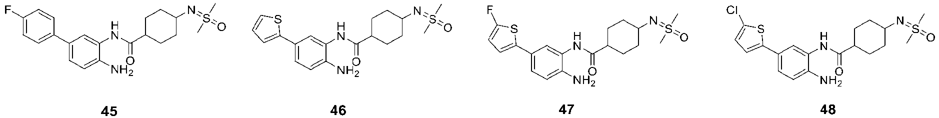

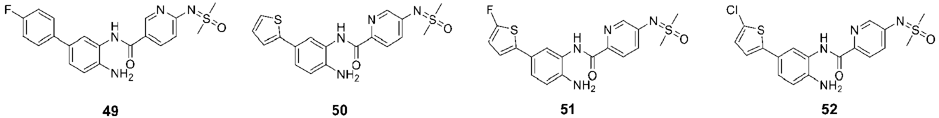

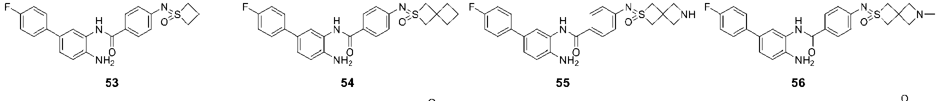

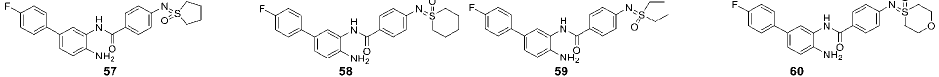

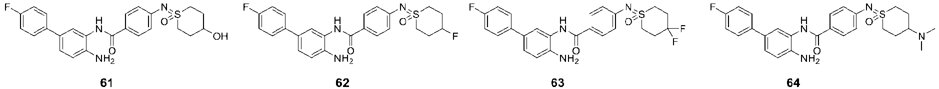

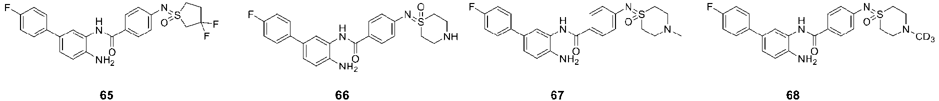

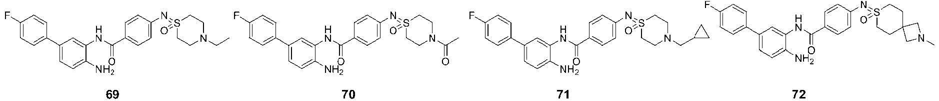

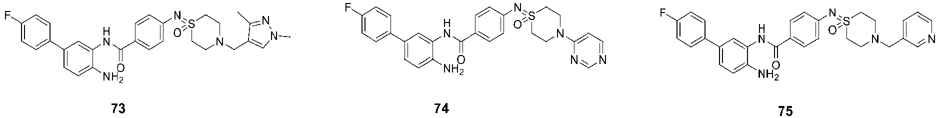

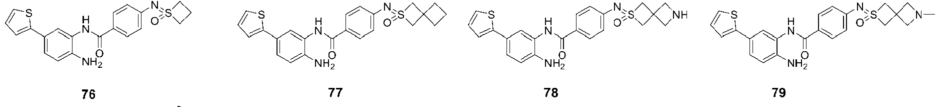

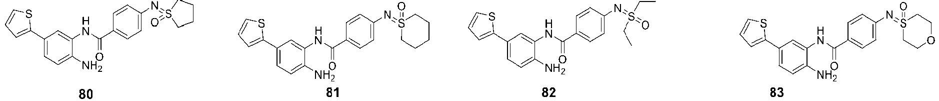

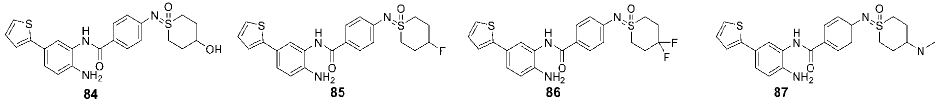

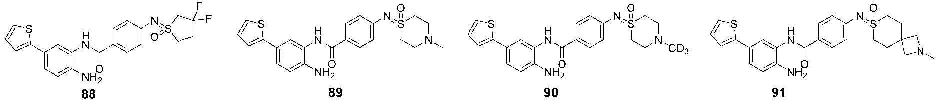

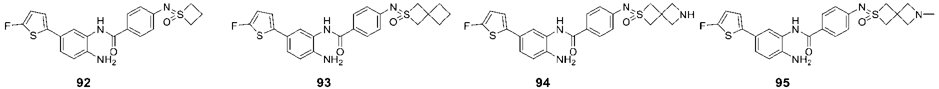

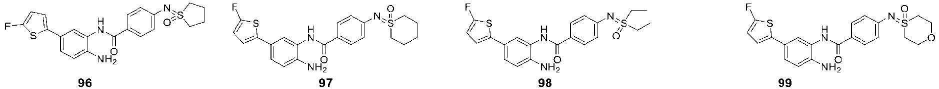

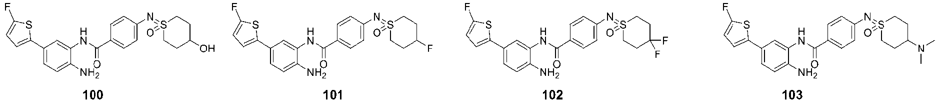

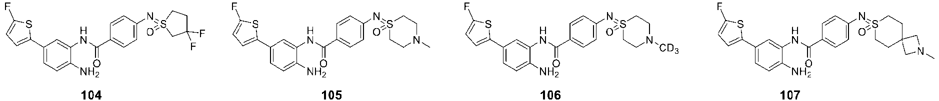

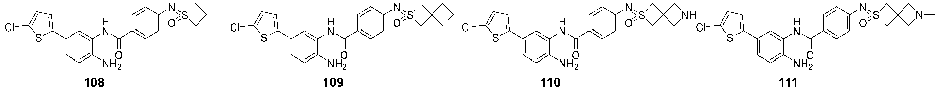

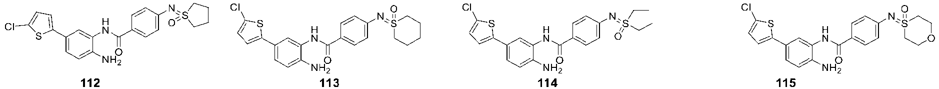

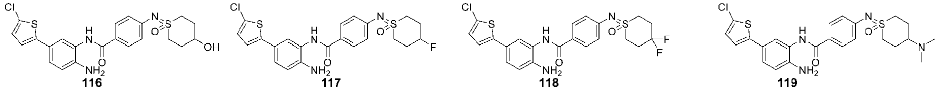

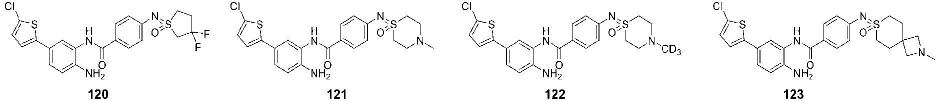

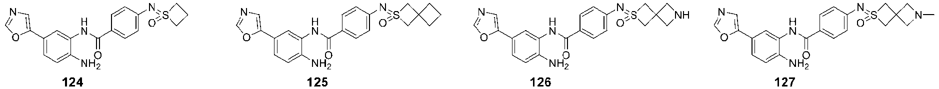

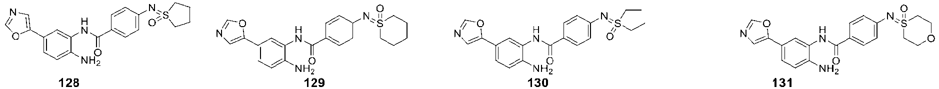

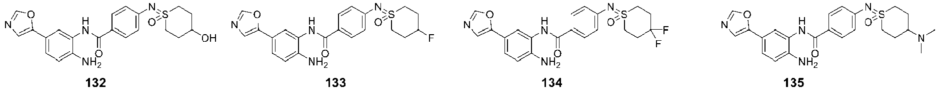

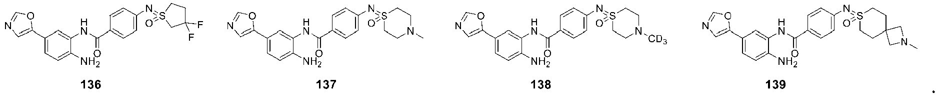

20. A pharmaceutical composition, comprising a pharmaceutically acceptable excipient or carrier, and the compound or the isomer thereof, the crystalline form thereof, the pharmaceutically acceptable salt thereof, the hydrate thereof, or the solvate thereof according to any one of claims 1 to 19 as an active ingredient.

21. A pharmaceutical composition, comprising a pharmaceutically acceptable excipient or carrier, and a therapeutically effective amount of the compound or the isomer thereof, the crystalline form thereof, the pharmaceutically acceptable salt thereof, the hydrate thereof, or the solvate thereof according to any one of claims 1 to 19, and a therapeutically effective amount of an immune checkpoint inhibitor as active ingredients.

22. A pharmaceutical composition, comprising a pharmaceutically acceptable excipient or carrier, and a therapeutically effective amount of the compound or the isomer thereof, the crystalline form thereof, the pharmaceutically acceptable salt thereof, the hydrate thereof, or the solvate thereof according to any one of claims 1 to 19, and a therapeutically effective amount of an immune checkpoint inhibitor and a therapeutically effective amount of a VEGFR inhibitor as active ingredients.

23. Use of the compound or the isomer thereof, the crystalline form thereof, the pharmaceutically acceptable salt thereof, the hydrate thereof, or the solvate thereof according to any one of claims 1 to 19 or the pharmaceutical compositions according to claims 20 to 22 in preparing a medicament for treating, regulating, and/or preventing an HDAC inhibitor-associated disease.

24. The use according to claim 23, wherein the disease is cancer, and the cancer is a hematologic cancer or a solid tumor.

25. The use according to claim 24, wherein the cancer comprises breast cancer, colon cancer, uterine cancer, pancreatic cancer, lung cancer, gastric cancer, leukemia, lymphoma, prostate cancer, liver cancer, cervical cancer, neuroblastoma, melanoma, or intracranial tumors.
